# EUROPEAN PATENT APPLICATION

(11) **EP 4 248 963 A1**
(43) Date of publication of application: **27.09.2023**
(21) Application number: 22000080.6
(22) Date of filing: 25.03.2022
(51) Int. Cl.: A61K 31/225, A61K 31/502, A61P 17/06, A61P 25/00, A61P 25/28

(54) **COMBINATION OF 5-AMINO-2,3-DIHYDRO-1,4-PHTALAZINEDIONE AND A FUMARIC ACID ESTER**

(71) Applicant: MetrioPharm AG, 8021 Zürich (CH)
(72) Inventor: The designation of the inventor has not yet been filed

(57) **Abstract**

The present application relates to a combination of 5-amino-2,3-dihydro-1,4-phthalazinedione or one of its pharmaceutically acceptable salts and at least one fumaric acid ester or a pharmaceutically acceptable salt thereof. In particular, the invention relates to the use of 5-amino-2,3-dihydro-1.4-phthalazinedione sodium salt In said pharmaceutical combination. Pharmaceutical compositions and advantageous formulation techniques are disclosed.

## Description

The present application relates to a combination of 5-amino-2,3-dihydro-1,4-phthalazinedione or one of its pharmaceutically acceptable salts and at least one fumaric acid ester or a pharmaceutically acceptable salt thereof. In particular, the invention relates to the use of 5-amino-2,3-dihydro-1,4-phthalazinedione sodium salt in said pharmaceutical combination. Pharmaceutical compositions and advantageous formulation techniques are disclosed,

### BACKGROUND OF THE INVENTION

Dimethyl fumarate (DMF) is a diester of fumaric acid and methanol. DMF and other fumaric acid esters are used as pharmaceutical agents in the treatment of multiple sclerosis and psoriasis.

DMF is an oral drug indicated for the twice-daily treatment in relapsing forms of multiple sclerosis (MS) and relapsing-remitting MS (RRMS). It displays immunomodulating and neuroprotective properties, both of which seem to contribute to its efficacy (Deeks (2016) Drugs 76: 243-254).

For the treatment of moderate-to-severe psoriasis DMF is marketed in the United Kingdom as monosubstance. In Germany, DMF is marketed for this indication as Fumaderm^{®}.

Fumaric acid esters show a good efficacy in these diseases. They are disease-modifying but cannot completely suppress the respective symptoms of the disease. It would therefore be desirable to increase the efficacy of fumaric acid esters.

Common adverse side effects of fumaric acid esters include gastrointestinal problems (diarrhea, nausea, stomach cramps, upper abdominal pain and flatulence), flushing, skin redness, skin irritation, headaches and lymphopenia. Less common adverse side effects in psoriasis treatment are progressive multifocal leukoencephalopathy and Fanconi syndrome. In multiple sclerosis treatment, seldomly anaphylaxis, angioedema, progressive multifocal leukoencephalopathy and liver damage occur (cf. Xu et al. (2015) The Cochrane Library Reviews 4 No. CD011076; Gieselbach et al. (2017) J Neurol 264: 1155-1164).

Further, DMF was used as a biocide against molds e.g., for protecting leather furniture or shoes. As there was an increasing number of reports of allergic reactions, DMF was banned in the European Union for this application. DMF has thus also a considerable allergic potential.

There is thus also a medical need for finding a formulation or composition that allows for reducing the administered amounts of fumaric acid esters while maintaining their therapeutic efficacy.

Surprisingly, these effects can be achieved by a pharmaceutical combination of 5-amino-2,3-dihydro-1,4-phthalazinedione or one of its pharmaceutically acceptable salts or solvates, hydrates and at least one fumaric acid ester or a pharmaceutically acceptable salt thereof. A combination of 5-amino-2,3-dihydro-1,4-phthalazinedione sodium salt with DMF shows an increase in the efficacy in the EAE (experimental autoimmune encephalopathy) model in mice, compared to DMF alone. Further, this combination allows for reducing the dosage of DMF and thus reduces the beforementioned adverse side effects. Supraadditive effects are observed.

Thus, the present application discloses a pharmaceutical combination of 5-amino-2,3-dihydro-1,4-phthalazinedione or one of its pharmaceutically acceptable salts, hydrates or solvates and at least one fumaric acid ester or a pharmaceutically acceptable salt thereof.

Especially, a pharmaceutical combination of 5-amino-2,3-dihydro-1.4-phthalazinedione or one of its pharmaceutically acceptable salts, hydrates or solvates and at least one fumaric acid ester or a pharmaceutically acceptable salt thereof for use in medicine is disclosed.

### DESCRIPTION OF THE INVENTION

5-amino-2,3-dihydro-1,4-phthalazinedione (luminol) belongs to the pharmaceutical class of the phthalazinediones. Compounds of this class are known for their beneficial antiinflammatory action. 5-amino-2,3-dihydro-1,4-phthalazinedione is also known under the name luminol. Luminol has excellent chemiluminescent properties. It is widely applied in diagnostic assays as a detection means and in forensic medicine, for example for tracing blood spots. In medicine, 5-amino-2,3-dihydro-1,4-phthalazinedione has been developed in the form of a sodium salt. In some countries it is approved for a broad range of acute and chronic inflammatory disorders for example of the intestinal tract, hepatitis B and C, gastroenteritis, inflammations such as prostatitis, endometriosis, throat inflammation, bronchial asthma, pneumonia, periodontitis, pyelonephritis and autoimmune diseases such as Crohn's disease, ulcerative colitis, lupus erythematosus and scleroderma. 5-amino-2,3-dihydro-1,4-phthalazinedione could effectively prevent cytokine storms caused by excessive immune reactions. Further, there is still a long list of indications in scientific and patent literature in the treatment of which 5-amino-2,3-dihydro-1,4-phthalazinedione sodium salt was allegedly tested or a beneficial use was suggested (cf. WO 2004/041169; WO 2007/018546; WO 2012/127441; WO 2016/096143; WO 2017/202496; WO 2018/082814: i.a.).

While most conventional immunomodulatory drugs show serious adverse reactions, or are at least problematic in long-term treatment, 5-amino-2,3-dihydro-1,4-phthalazinedioneand its pharmaceutically acceptable salts are well tolerated and have a high safety margin in respect to administered dosages.

To ensure a better solubility and bioavailability pharmaceutically acceptable salts of 5-amino-2,3-dihydro-1,4-phthalazinedione are used. Sodium, potassium and lithium salts have been described for therapeutic applications (cf. WO 2010/082858). Crystal structures for lithium, sodium, potassium, rubidium and cesium salts were described in Guzei et al. (2013) Journal of Coordination Chemistry 66, 3722-3739. Thus, the present patent application refers also to the use of all pharmaceutically acceptable salts of 5-amino-2,3-dihydro-1,4-phthalazinedione.

Fumaric acid esters (FAE), also known as fumarates, are ester derivatives of fumaric acid. Fumaric acid is an intermediate in the citric acid cycle, a basic cellular process for generating energy in the mitochondria. Given that oral fumaric acid causes too much gastrointestinal irritation esters of fumaric acid were developed for pharmaceutic uses (cf. Balak (2015) Psoriasis 5: 9-23). Dimethyl fumarate (DMF) is by far the most widely used fumaric acid ester. DMF is a methyl ester of fumaric acid. As an α,β-unsaturated carboxylic ester generated by reacting fumaric acid with methanol in the presence of sulfuric acid, DMF notably stimulates mitochondrial tricarboxylic acid cycle activity and ATP production (Landeck et al. (2018) Arch Dermatol Res 310: 475-483). While fumaric acid is poorly absorbed in the gastrointestinal tract DMF and its active metabolite monomethyl fumarate (MMF) display a robust bioavailability and exert beneficial effects in inflammation, neurodegeneration and toxic oxidative stress (cf. Kourakis et al. (2020) Pharmaceuticals 13: 306). Recently, diroximel fumarate (DRF) has been developed showing a comparable efficacy but with less side effects (Naismith (2020) CNS Drugs 34: 185-196). The mode-of-action of FAE seems to be mediated by the activation of Nrf2 (nuclear erythroid 2-related factor), a transcription factor for upregulating the transcription of a battery of anti-oxidative and Phase II cytoprotective genes. The Nrf2 pathway is considered a critical cellular defense system against potentially toxic stimuli. Another effect of FAE is the interaction with the antiinflammatory hydroxycarboxylic acid receptor 2 (HCAR2) (Parodi et al. (2015) Acta Neuropathol 130: 279-295); Chen et al. (2014) J Clin Investig 124: 2188-2194).

In the treatment of relapsing forms of multiple sclerosis (MS) and relapsing-remitting MS (RRMS) DMF is used. In the treatment of moderate-to-severe psoriasis DMF is used in the United Kingdom while in Germany a mixture of DMF and MMF salts are used (calcium, magnesium and zinc salts). Ethyl hydrogen fumarate is a synonym of MMF.

Pharmaceutically acceptable salts should be seen in terms of this application as an active agent containing a compound according to the invention in form of a salt, in particular if this salt bestows specific or ameliorated pharmacokinetic properties in comparison to the free form of the active agent or to another salt of the active agent. The pharmaceutically acceptable salt of the active agent may also bestow a pharmacokinetic characteristic to the active agent it did not have in its free form. Thus, it may even positively influence the pharmacodynamics of the active agent in respect to its therapeutic efficacy in the organism.

It is therefore desirable to find a medication that allows for boosting the effects of fumaric acid esters or a pharmaceutically salt thereof for increasing the therapeutic efficacy in the treatment of multiple sclerosis, psoriasis and related disorders. A further desirable action would be that the administered dosage of the fumaric acid ester or at least one of its pharmaceutically acceptable salts could be reduced for avoiding or at least mitigating adverse side effects associated with them. It is reasonable to assume that by reducing the dosage of a fumaric acid ester or at least one of its pharmaceutically acceptable salts through a pharmaceutical combination according to the disclosure also their adverse side effects are reduced.

To avoid ambiguities in the art, the following terms of interaction among drugs are defined. They are used in this sense throughout the disclosure. If at least two substances (e.g., pharmaceutical agents) are administered concomitantly and at least one of these substances affects the activity of the other at least one substance a so-called drug-drug interaction is given. If said interaction leads to an exaggerated or increased effect of the drug substance the effect is synergistic. The respective equation can be displayed as (A+B) > A or (A+B) > B, wherein A and B are the percentual or fractional effects (i.e., a value between 0 and 1) seen when the respective substances are administered alone and (A+B) is the percentual or fractional effect seen after combinatory application, respectively. A final effect equal to the sum of the effects seen in the single drug applications is an additive synergistic effect. If the final effect is greater than said expected effect the term supraadditivity is used. Smaller effects are subadditive. If, on the other hand the drug-drug interaction leads to a decreased effect of the drug substance the effect is not synergistic at all, but antagonistic.

Hence, in the scope of this disclosure the terms "antagonism" and "antagonistic" are used for any decreased drug effect induced by drug-drug-interaction, whilst the terms "synergy" and "synergistic" are used for any increased drug effect induced by drug-drug-interaction. To describe and determine the grade of said synergy the terms "subadditive", "additive" and "supraadditive" as well as the respective nouns are used. Hence, the term supraadditivity is used to describe an effect of two or more combined agents that is greater than the expected additive effects of the single agents.

The use of synergistic acting drug combinations can both help to increase the therapeutic efficacy and the potency, wherein the latter primarily helps to reduce off-target toxicity.

The identification of drug-drug-interactions depends on the "no-interaction" null-hypotheses, which is based on the observed drug response and not on any model of mechanism. Hence, for two different drugs, their grade of additivity is based on a point of reference for the readout wherein the point of reference depends on the mathematical model chosen. Various methods exist to identify such interactions and to calculate the expected additive effect of two substances and thus to determine if the actual synergistic effect observed is subadditive, additive or supraadditive. Different methods are recommended in the art. The most common methods are outlined below.

Basic methods like building simple arithmetic sums or the fractional product method provide an easy way to gain first insights if a specific combination has supraadditive potential.

The simple arithmetic sum method for additivity is based on the equation A + B = (A+B), wherein A and B again are the percentual or fractional effects seen when the respective substances are administered alone and (A+B) is the percentual or fractional effect seen after combinatory application of said substances using the same doses as in the single applications. If A + B > (A+B) supraadditivity is given. An obviously striking disadvantage of this method is that results wherein A + B ≥ 100% cannot be analyzed for supraadditivity. Hence, this method is primarily used to interpret combinations of suboptimal doses.

The fractional product method is based on the equation 1 - (1-A)·(1-B) = (A+B). Here, A and B are the fractual effects seen when the respective substances are administered alone and (A+B) is the fractual effect seen after combinatory application of said substances using the same doses as in the single applications. If 1 - (1-A)·(1-B) > (A+B), supraadditivity is given. More sophisticated methods include the use of a so-called isobologram, a graph constructed on a coordinate system defined by individual drug doses showing a "line of additivity" that allows to distinguish between subadditive, additive (along the line) and supraadditive effects. The "line of additivity" connects those single drug doses which display the same effect (e.g., 50% inhibition of a specific marker). All possible dose combinations along this line are expected to show the same effectiveness. Dose combinations located within the triangle built by coordinates and the line of additivity i.e., nearer to the arbitrary point, displaying the same effect are considered as supraadditive. Dose combinations located outside the triangle are considered as subadditive. Respective figures can be mapped using specific software as e.g., CompuSyn (Chou TC and Martin N. ComboSyn, Inc. Paramus, NJ 2007 [www.combosyn.com]).

Also, further specific indicator values as for example the Combination Index (CI. equation of Chou and Talalay (1984) Adv Enzyme Reg 22: 27-55) and the Dose-Reduction Index (DRI, Chou equation 1984) can be calculated easily using the beforementioned specific software. Both these values allow to determine if drug substances act supraadditive synergistically when administered contemporarily. The CI is based on the principles of mass action law and is applicable for any kind of drug combination regardless of mechanism of action, dynamic order or the quantity units used for each drug in the combination. The CI value defines synergism as supraadditive if CI<1 and as additive if CI =1. If CI > 1 the effect is either subadditive or antagonistic. Hence, a CI of 1 also refers to the "line of additivity" In the classic isobologram as mentioned above. In a simplified approach it could be calculated as follows: CI = A(t)/A(x) + B(t)/B(x), wherein A(t) respectively B(t) is the dose of drug A respectively B alone that inhibits x %, whereas A(x) and B(x) stand for the portion of the respective drug in the combination that also inhibits x %. The DRI is a measure of how many folds the dose of each drug in a synergistic combination may be reduced at a given effect level compared with the doses of each drug alone. Therefore, DRI=1 indicates additivity, whereas DRI>1 and <1 indicate supraadditivity and subadditivity (or antagonism), respectively. For displaying purposes, the isobologram, i.e., the equi-effective curve at various concentrations or doses of two drugs as mentioned above, is a dose-oriented figure approach based on a special case of the CI equation. A more convenient figure approach, however, is the effect oriented so-called FaCI plot, displaying the Combination Index (CI) against the fractual effect (Fa), preferably for a specific dose combination.

Multiple sclerosis (MS) is a generic term for the most frequent demyelinating disease of the central nervous system (CNS) in humans. The disease pattern is highly variable among patients. There are, however, two main variants that differ widely in their pathophysiology and their clinical manifestation. The first variant is relapsing-remitting multiple sclerosis (RRMS) which accounts for roughly 80% of all incidences, and the second variant are the chronic progressive forms of multiple sclerosis which are subdivided into primary progressive multiple sclerosis (PPMS) and secondary progressive multiple sclerosis (SPMS). In the International Statistical Classification of Diseases and Related Health Problems 10th Revision (ICD-10-GM, German Modification, Version 2017, DIMDI 23Sep2016) RRMS, PPMS and SPMS are classified as G35.1, G35.2 and G35.3, respectively. PPMS accounts for approximately 10 to 20% of all MS cases. There is an ongoing controversy whether RRMS and PPMS/SPMS are actually two different diseases that only coincidentally may become manifest together sharing a number of common pathophysiological features but are clearly differing in others.

RRMS is characterized by the incidence of unpredictable acute exacerbations (relapses) in the disease course, followed by phases of recovery to the previous base disease level, often of full recovery (remission). The intervals between relapses usually become shorter the longer a person is suffering from this disease. In most cases RRMS starts with a clinically isolated syndrome (CIS). A CIS represents an attack in one organ system probably due to demyelination. 30 to 70% of persons experiencing a CIS attack later develop MS.

Beneficial effects of 5-amino-2,3-dihydro-1,4-phthalazinedione sodium salt on RRMS have been postulated e.g., in WO 2011/107295 A1.

PPMS is characterized clinically by the accumulation of neurological disability without unequivocal recovery (as reviewed by Pérez Cerdá et al. (2016) Multiple Sclerosis and Demyelinating Disorders 1:9). The disease progress in PPMS is more insidious and subtle than in RRMS with clearly defined relapses. There is a characteristic neuronal atrophy due to Wallerian-like degeneration, caused by demyelination and neurodegenerative oxidative damage of the axon. The characteristic onset of clinical symptoms in PPMS patients is around 40 years of age. PPMS is disproportionally more frequent in relatively MS-resistant populations (Africans, Orientals) and disproportionally more frequent in men. Whilst in general the relation of women and men suffering from MS is about 3 to 1 the relation in PPMS is approximately 1 to 1.

SPMS is diagnosed after a previous RRMS stops developing relapse periods over a minimum of 3 months and a continuous deterioration of the clinical symptoms occurs. The symptoms of SPMS are substantially the same as of PPMS, although they may differ quantitatively. Therefore, most clinicians regard SPMS and PPMS as the same disease, mainly differing whether there was a previous phase of RRMS or not. The age difference concerning the onset of RRMS and PPMS is typically about 10 years, however the switch from RRMS to SPMS occurs at approximately the same age as PPMS usually becomes manifest (as reviewed by Antel et al. (2012) Acta Neuropathol 123:627-638).

Beneficial effects of 5-amino-2,3-dihydro-1,4-phthalazinedione sodium salt on PPMS and SPMS could be shown in WO 2018/082814 A1.

Another group of neurodegenerative diseases related to multiple sclerosis are the so-called white matter diseases (WMD). In the scope of the present application they will be also referred to as multiple-sclerosis-related WMD affect the pattern of normal myelination and include a large diversity of congenital and acquired processes. They can be divided into primary and secondary types according to their cause. Primary demyelinating disorders are of unknown etiology e.g., multiple sclerosis. Secondary demyelinating diseases include a variety of known causes. The underlying condition is damage to the myelin sheath or to myelin-producing cells such as oligodendrocytes. The combining link of the effects of the pharmaceutical combination according to the disclosure in the EAE (experimental autoimmune encephalomyelitis) model is the brain histology of the EAE mice after ending the experiment. As can be seen in the Examples, the pharmaceutical combination according to the disclosure could diminish the characteristic invasion / infiltration of leukocytes into the CNS, forming typical nuclei of demyelinated axons (corresponding to a loss of white matter) that can be identified either by staining or by radiologic methods. As this loss of white matter is a common feature of WMD, it is reasonable to assume that the pharmaceutical combination according to the disclosure as well as 5-amino-2,3-dihydro-1,4-phthalazinedione sodium salt alone display also beneficial effects on WMD.

Typical WMD include:
- Acute disseminated encephalomyelitis (ADEM) is a rare autoimmune disease marked by a sudden widespread attack of inflammation in the CNS. The myelin insulation os damaged and in consequence the white matter. It is often triggered by a viral infection (cf. Garg (2003) Postgrad Med J 79: 11-17).
- Neuromyelitis optica (NMO; synonym: Devic's disease) and neuromyelitis optica spectrum disorders (NMOSD): They are predominantly characterized by acute inflammation of the optic nerve. They are caused by immunoglobulin G autoantibodies to aquaporin 4 (anti-AQP4), the most abundant water channel protein in the CNS (Lana Peixoto et al. (2019) Biomedicines 7: 42).
- Idiopathic inflammatory demyelinating disease are variants of multiple sclerosis
- Central pontine myelinolysis is a neurological disorder involving severe damage to the myelin sheath of neurons in the pons. It is mainly iatrogenic and is characterized by acute paralysis, dysarthria and dysphagia (Yoon et al. (2008) Alcohol 43: 647-649).
- Tabes dorsalis (syphilitic myelopathy) is a sequela of neurosyphilis, characterized by a slow neuronal generation in the dorsal root ganglia. Patients have lancinating neural pain which comes along with cough and sensory ocular ataxia.
- Progressive multifocal leukoencephalopathy (PML) is a rare and often fatal viral disease (infections with the JC virus, human polyomavirus 2). PML is characterized by progressive damage or inflammation of the white matter in the CNS at multiple locations. PML becomes manifest almost exclusively in patients with severe immune deficiency and in these patients a mortality rate of 30-50% is observed.
- Acute hemorrhagic leukoencephalopathy (AHL, Hurst's disease) is a hyperacute and often fatal form of ADEM. It is characterized by necrotizing vasculitis of venules, hemorrhage and edema.
- Toxic leukoencephalopathy is a rare disease characterized by progressive damage to the white matter in the CNS due to exposure of drug use, environmental toxins or chemotherapeutic drugs (Filley and Kleinschmidt-de Masters (2001) N Engl J Med 345: 425-432).
- Leukoencephalopathy with vanishing white matter (VWM disease) is an autosomal recessive neurological disease. The cause are gene mutations in the translation initiation factors eIF2B, EIF2B2, IF2B3, EIF2B4 or EIF2B5. Symptoms include cerebellar ataxia, spasticity, optic atrophy, epilepsy loss of motor functions irritability, vomiting and coma (van der Knaap et al. (2006) Lancet Neurology 5: 413-423).
- Leukoencephalopathy with neuroaxonal spheroids (LENAS) is a very rare idiopathic neurodegenerative leukoencephalopathy. LENAS causes severe and subacute dementia. White matter damage results from swellings which are termed spheroids.
- Reversible posterior leukoencephalopathy syndrome (PRES, reversible posterior leukoencephalopathy syndrome) is a rare disease in which swelling occurs on parts of the brain, usually secondary to another condition such as severely elevated blood pressure, kidney failure, severe infections or pre-eclampsia. Symptoms include headache, visual disorders, seizures, confusion or weakness in the extremities.
- Megalencephalic leukoencephalopathy with subcortical cysts (MLC, van der Knaap disease) is a hereditary CNS demyelinating disease. Causes can be mutations in the MLC1, MLC2A or MLC2B genes (cf. van der Knaap et al. (1996) Acta neuropath 92: 206-212).
- Hypertensive leukoencephalopathy is a disease characterized by a degeneration of the white matter in the CNS following a sudden increase in blood pressure. Further symptoms include acute confusional state headaches, vomiting and seizures, possibly also retinal hemorrhages and hard exudates.
- Metachromatic leukodystrophy (MLD) is a lysosomal storage disease that affects the metabolism of sphingolipids. Leukodystrophies impair the growth and/or development of myelin. An autosomal recessive inheritance pattern was identified in MLD.
- Krabbe disease (KD, globoid cell leukodystrophy, galactosylceramide lipidosis) is a rare severe lysosomal storage disease that results in progressive damage to the nervous system. Sphingolipoid metabolism is impaired. The inheritance pattern is autosomal recessive. Symptoms include irritability, fever, limb stiffness, seizuresm feeding difficulties, vomiting, staring episodes and a retarded cognitive and motor development (Wasserstein and Andriola (2016) Genetics in Medicine 18: 1236-1243).
- Canavan disease is a fatal autosomal recessive neurodegenerative disease that causes a progressive loss of white matter in the CNS. The underlying reason is a deficiency is aminoacyclase 2 (ASPA). Symptoms appear in early infancy, typically Intellectual disability, loss of previously acquired motor skills, feeding difficulties, abnormally low muscle tone, poor, head control, megalocephaly and sometimes paralysis, blindness or seizures (cf. Kumar et al. (2006) Mental Retardation and Developmental Disabilities Research Reviews 12: 157-165).
- Alexander disease is a very rare autosomal dominant leukodystrophy with an abnormal myelin, above all in infants. Symptoms include cognitive and physical development delay. An abnormal increase in head size and seizures, further excessive vomiting, swallowing and speaking difficulties, poor coordination and loss of motor control. The underlying cause is a defect in the GFAP (glial fibrillary acidic protein) gene.
- Adrenomyeloneuropathy (ALD, X-linked adrenoleukodystrophy) is an X-chromosome-linked disease (ABCD1 gene). It results from fatty acid buildup caused by a defect In the chain of fatty acid transporters in peroxisomes, leading to a damaged myelin sheath of neuronal axons. Symptoms in infancy include emotional instability, hyperactivity and disruptive behavior. In the adult age typical symptoms are muscle stiffness, paraparesis and sexual dysfunction (cf. Moser et al. (2007) Nature Clin Pract Neurol 3: 140-151).
- Cerebrotendineous xanthomatosis (CTX, cerebral cholesterosis) in an autosomal recessive form of xanthomatosis, due to mutations in the CYP27A1 gene. It is characterized by a cerebellar ataxia and by juvenile cataracts, chronic diarrhea neurological deficits and tendineous or tuberous xanthomas (Pilo de la Fuente et al. (2008) J Neurol 255: 839-842).
- Pelizaeus-Merzbacher disease is an X-linked neurological disorder that damages oligodendrocytes. It is due to mutations in the PLP1 (proteolipid protein 1) gene that largely contributes to myelin formation. Hallmarks are little or no movement of the extremities, respiratory difficulties and characteristic left-to-right movements of the eye. Initial symptoms include nystagmus and low muscle tone (Hobson and Garbern (2012) Seminars in Neurology 32: 62-67).
- Hypomyelinating leukodystrophy type 7 (4H syndrome, HLD7) is caused by autosomal recessive mutations of the POLR3A gene which lead to a hypomyelinization of axons. Symptoms include progressive motor decline, spasticity, ataxia, tremor, cerebellar symptoms, mild cognitive regression and hypodontia (cf. Wu et al. (2019) BMC pediatrics 19; 289).
- Refsum disease is an autosomal recessive disorder that results in over-accumulation of phytanic acid in cells and tissues. The underlying cause is an impaired alpha-oxidation of branched fatty acids due to mutations in the PEX7 gene. Patients present with neurologic damage, cerebellar degeneration and peripheral neuropathy. Further symptoms include ataxia, ichthyosis, hearing difficulties and ophthalmologic problems such as retinitis pigmentosa, cataracts and night blindness (cf. Masuhr (2013) Akt Neural 40: 58).
- Tumefactive demyelinating lesions occur in persons with demyelinating lesions with atypical characteristics of standard multiple sclerosis
- In Baló concentric sclerosis the white matter damage in the brain appears in concentric layers, leaving the axis cylinder intact. This is a borderline form of multiple sclerosis.
- Marburg multiple sclerosis (acute fulminant multiple sclerosis) belongs to the multiple sclerosis borderline diseases. The ultrarapid disease course can lead to severe disabilities during days and to death after several months.
- Schilder's disease (diffuse myelinoclastic sclerosis) is another borderline form of multiple sclerosis characterized by pseudotumoural demyelinating lesions with plaques larger than 2 cm diameter.
- Solitary sclerosis refers to stand-alone tumefactive demyelinating lesions larger than 2 cm looking like intracranial neoplasms.
- Myelocortical multiple sclerosis is a variant of multiple sclerosis with demyelination in the spinal cord and cerebral cortex but not of cerebral white matter.
- Optic-spinal multiple sclerosis (OSMS) is characterized by the selective and severe involvement of the optic nerves and the spinal cord in a multiple sclerosis disease pattern (cf. Fujihara (2006) Rinsho Shinkeigaku 46: 866-868).
- The features of pure spinal multiple sclerosis are suggestive of spinal cord involvement of multiple sclerosis but without other typical CNS signs.
- LHON (Leber's hereditary optic neuropathy) associated multiple sclerosis causes multiple sclerosis-like CNS damage. Demyelination leads to an acute or subacute loss of central vision.
- Oligoclonal band negative multiple sclerosis is a variant of multiple sclerosis in which a immunochemically stained IgG band is absent (Zeman et al. (1996) J Neurol Neurosurg Psych 60: 27-30).
- Oligoclonal IgM positive multiple sclerosis is a variant of multiple sclerosis in which additionally to IgG also IgM are found intrathecally (cf. Monreal et al. (2021) Neurol Neuroimmun Neuroinflamm 8: 5).
- Lyme disease is a vector-bome disease caused by *Borrelia* bacteria. It can cause a chronic encephalomyelitis that resembles multiple sclerosis. It is progressive and can entail cognitive impairment, brain fog, migraines, balance problems, weakness in the legs, awkward gait, facial palsy, bladder problems, vertigo and back pain.
- Human immunodeficiency virus encephalopathy is a neurocognitive disorder due to a HIV infection and is associated with a metabolic encephalopathy and fueled by immune activation of macrophages and microglia (Gray et al. (2001) Clin Neuropathol 20: 146-155).

In another aspect the present application refers thus to a pharmaceutical combination of 5-amino-2,3-dihydro-1,4-phthalazinedione or one of its pharmaceutically acceptable salts, hydrates or solvates and at least one fumaric acid ester or a pharmaceutically acceptable salt thereof for use in the prophylaxis or treatment of relapsing-remitting multiple sclerosis, primary progressive multiple sclerosis, secondary progressive multiple sclerosis, acute disseminated encephalomyelitis, neuromyelitis optica, idiopathic inflammatory demyelinating diseases, central pontine myelinolysis, tabes dorsalis, progressive multifocal leukoencephalopathy, acute hemorrhagic leukoencephalopathy, toxic leukoencephalopathy, leukoencephalopathy with vanishing white matter, leukoencephalopathy with neuroaxonal spheroids, reversible posterior leukoencephalopathy syndrome, megalencephalic leukoencephalopathy with subcortical cysts, hypertensive leukoencephalopathy, metachromatic leukodystrophy, Krabbe disease, Canavan disease, Alexander disease, cerebrotendineous xanthomatosis, Pelizaeus-Merzbacher disease, hypomyelinating leukodystrophy type 7, Refsum disease, tumefactive demyelinating lesions, Bald concentric sclerosis, Marburg multiple sclerosis, Schilder's disease, solitary sclerosis, myelocortical multiple sclerosis, optic-spinal multiple sclerosis, pure spinal multiple sclerosis, LHON associated multiple sclerosis, oligoclonal band negative multiple sclerosis, oligoclonal IgM positive multiple sclerosis, Lyme disease and human immunodeficiency virus encephalopathy.

In the scope of the present disclosure these diseases will be subsumed under the terms "multiple sclerosis-type diseases" or "multiple sclerosis-related disorders".

As can be seen in the Examples, 5-amino-2,3-dihydro-1,4-phthalazinedione sodium salt alone is also effective in the EAE model indicative for multiple sclerosis.

The present disclosure refers therefore also to 5-amino-2,3-dihydro-1,4-phthalazinedione or one of its pharmaceutically acceptable salts, hydrates or solvates in the prophylaxis or treatment of demyelinating diseases which are selected from a group consisting of acute disseminated encephalomyelitis, neuromyelitis optica, idiopathic inflammatory demyelinating diseases, central pontine myelinolysis, tabes dorsalis, progressive multifocal leukoencephalopathy, acute hemorrhagic leukoencephalopathy, toxic leukoencephalopathy, leukoencephalopathy with vanishing white matter, leukoencephalopathy with neuroaxonal spheroids, reversible posterior leukoencephalopathy syndrome, megalencephalic leukoencephalopathy with subcortical cysts, hypertensive leukoencephalopathy, metachromatic leukodystrophy, Krabbe disease, Canavan disease, Alexander disease, cerebrotendineous xanthomatosis, Pelizaeus-Merzbacher disease, hypomyelinating leukodystrophy type 7, Refsum disease, tumefactive demyelinating lesions, Baló concentric sclerosis, Marburg multiple sclerosis, Schilder's disease, solitary sclerosis, myelocortical multiple sclerosis, optic-spinal multiple sclerosis, pure spinal multiple sclerosis, LHON associated multiple sclerosis, oligoclonal band negative multiple sclerosis, oligoclonal IgM positive multiple sclerosis, Lyme disease and human immunodeficiency virus encephalopathy.

In other words, the present disclosure refers to 5-amino-2,3-dihydro-1,4-phthalazinedione or one of its pharmaceutically acceptable salts, hydrates or solvates in the prophylaxis or treatment of white matter diseases which are selected from a group consisting of acute disseminated encephalomyelitis, neuromyelitis optica, idiopathic inflammatory demyelinating diseases, central pontine myelinolysis, tabes dorsalis, progressive multifocal leukoencephalopathy, acute hemorrhagic leukoencephalopathy, toxic leukoencephalopathy, leukoencephalopathy with vanishing white matter, leukoencephalopathy with neuroaxonal spheroids, reversible posterior leukoencephalopathy syndrome, megalencephalic leukoencephalopathy with subcortical cysts, hypertensive leukoencephalopathy, metachromatic leukodystrophy, Krabbe disease, Canavan disease, Alexander disease, cerebrotendineous xanthomatosis, Pelizaeus-Merzbacher disease, hypomyelinating leukodystrophy type 7, Refsum disease, tumefactive demyelinating lesions, Baló concentric sclerosis, Marburg multiple sclerosis, Schilder's disease, solitary sclerosis, myelocortical multiple sclerosis, optic-spinal multiple sclerosis, pure spinal multiple sclerosis, LHON associated multiple sclerosis, oligoclonal band negative multiple sclerosis, oligoclonal IgM positive multiple sclerosis, Lyme disease and human immunodeficiency virus encephalopathy.

Psoriasis is a chronic noncontagious autoimmune disease characterized by raised abnormal skin. These areas are red or purple, dry, itchy and above all scaly. The size of these areas can vary from small, localized patches to complete body coverage (cf. Boehncke and Schön (2015) Lancet 386: 983-994). No cure is known for psoriasis, but a number of symptomatic treatment options including monoclonal antibodies and fumaric acid esters.

Plaque psoriasis typically becomes manifest as raised areas of inflamed skin covered with silvery-white, scaly skin. These areas are called plaques and are most commonly found on the elbows, knee, scalp and back (Palfreeman et al. (2013) Drug Design Devel Ther 7: 201-210).

Pustular psoriasis is characterized by raised bumps filled with noninfectious pus. The skin under and surrounding the pustules is red and tender, it is often localized to the hands and feet (cf. Raychauduri et al. (2014) Autoimmun Rev 13; 490-495).

Inverse psoriasis (flexural psoriasis) becomes manifest as smooth, inflamed skin patches. They are often found in skin folds such as the genitals, armpits, panniculus, between the buttocks and below the breasts in the inflammatory fold (cf. Weigle and McBane (2013) Am Family Physician 87: 627-633).

Napkin psoriasis occurs typically in infants. Red papules with silvery scales appear in the diaper area that may extend to the torso or the limbs.

Guttate psoriasis is characterized by numerous small, scaly, red or pink, droplet-like papules that appear over large areas of the trunk, but also the limbs and scalp. There may be an underlying streptococcal infection (cf. Weigle and McBane (2013) Am Family Physician 87: 627-633).

Erythrodermic psoriasis is characterized by a widespread inflammation and exfoliation of the skin, often more than 90% of the body surface area. It is often an exacerbation of plaque psoriasis (Rendon and Schäkel (2019) Int J Mol Sci 20: 1475).

Seborrheic-like psoriasis is a form of psoriasis that combines symptoms of seborrheic dermatitis. It often occurs at the scalp. Forehead, skin folds next to the nose, around the mouth, above the sternum and in skin folds.

Psoriatic arthritis is a chronic inflammatory autoimmune disorder of the joints from the group of spondyloarthropathies. It frequently occurs in association with skin and nail psoriasis. Symptoms are painful inflammation of the joints and the surrounding connective tissue, mostly in the fingers and toes (Goldenstein-Schainberg et al. (2012) Rev Brasil Reumatol 52: 98-106). It shares a variety of symptoms with rheumatoid arthritis but has additionally some independent characteristics. Forms of psoriasis may occur at the same time. The etiology is unknown until now. All joints can be affected, but typically the distal and interphalangeal joints of the hands and the feet as well as the knees and the sacroiliac joints. The disease course is often asymmetric.

In another aspect the present application refers thus to a pharmaceutical combination of 5-amino-2,3-dihydro⁻1,4-phthalazinedione or one of its pharmaceutically acceptable salts, hydrates or solvates and at least one fumaric acid ester or a pharmaceutically acceptable salt thereof for use in the prophylaxis or treatment of psoriasis, plaque psoriasis, pustular psoriasis, inverse psoriasis, napkin psoriasis, guttate psoriasis, erythrodermic psoriasis, seborrheic-like psoriasis and psoriatic arthritis.

In the scope of the present disclosure these diseases will be subsumed under the term "psoriasis-type diseases" or "psoriasis-like disorders".

5-amino-2,3-dihydro-1,4-phthalazinedione is often used as a hydrate, for example as sodium salt dihydrate. Thus, the present patent application refers also to the use of all hydrates and other solvates of 5-amino-2,3-dihydro-1,4-phthalazinedione and its pharmaceutically acceptable salts. 5-amino-2,3-dihydro-1,4-phthalazinedione or one of its pharmaceutically acceptable salts may build complexes with suitable ligands. Thus, the present patent application refers also to such complexes.

For ensuring a reproducible and standardized API production and to provide improved stability features of an active agent anhydrous formulations are often preferred. Anhydrate forms of 5-amino-2,3-dihydro-1,4-phthalazinedione sodium salt have been described as crystalline polymorphs in WO 2011/107295 (Form I, Form II) and WO 2016/096143 (Form III). These crystalline polymorphs are virtually free from phase impurities and were characterized by means of X-ray powder diffraction. This method yields a set of characteristic d-values indicating interplanar spacings [Å] and of the corresponding 2-theta (2θ) angles [°] under which Bragg reflections occur. This yields a unique and unambiguous fingerprint of the respective polymorphs.

For Form I the following values were determined:
d values: 13.5; 6.9; 5.2; 4.6; 3.9; 3.5; 3.4; 3.3; 3.1; 3.0 and/or
2-theta values: 6.5; 12.7; 16.9; 19.3; 22.8; 25.8; 26.6; 27.2; 28.7; 30.3.

Form II is characterized by the following values:
d values: 12.9; 7.9; 7.1; 6.5; 5.3; 4.0; 3.7; 3.6; 3.3; 3.2 and/or
2-theta values: 6.8; 11.2; 12.5; 13.7; 16.7; 22.4; 24.3; 24.9; 27.2; 27.8.

Form III yielded the following values:
d values: 13.131; 7.987; 7.186; 6.566; 6.512; 5.372; 3.994; 3.662; 3.406; 3.288; 3.283; 3.222; 3.215; 3.127; 2.889 and/or
2-theta values: 6.73; 11.07; 12.31; 13.48; 13.59; 16.49; 22.24; 24.29; 26.14; 27.10; 27.14; 27.67; 27.72: 28.52; 30.93.

The use of anhydrous Form I of 5-amino-2,3-dihydro-1,4-phthalazinedione sodium salt is preferred.

5-amino-2,3-dihydro-1,4-phthalazinedione itself shows also polymorphism. A Form I (Paradies (1992) Ber. Bunsen-Ges. Phys. Chem 96: 1027-1031) and a Form II (WO 2017/140430) have been disclosed.

WO 2017/140430 discloses also that 5-amino-2,3-dihydro-1,4-phthalazinedione has a great potential in the immunomodulatory treatment of inflammatory and autoimmune diseases. Crystalline Form II is particularly useful in the treatment of inflammatory and autoimmune respiratory diseases such as upper and lower respiratory tract infections.

Thus, the present patent application refers also to the use according to the invention of all crystalline forms and polymorphs thereof of 5-amino-2,3-dihydro-1,4-phthalazinedione and its pharmaceutically acceptable salts, hydrates and solvates. The use of Form II of 5-amino-2,3-dihydro-1,4-phthalazinedione is preferred.

Similar therapeutic effects are known for a variety of phthalazinediones, respectively of derivatives of 5-amino-2,3-dihydro-1,4-phthalazinedione and its pharmaceutically acceptable salts. An example is 6-amino-2,3-dihydrophthalazine-1,4-dione (isoluminol). An overview of suitable phthalazinediones is given in WO 2007/018546. It is reasonable to assume that these compounds show comparable effects when being used for the therapeutic applications according to the invention.

Tautomerism relates to a rapid intraconversion of organic compounds in which a hydrogen atom or proton formally migrates inside the compound. This is accompanied by a switch of a single bond and adjacent double bond. The single forms are called tautomers. For example, keto-enol tautomerism occurs in 5-amino-2,3-dihydro-1,4-phthalazinedione (Proescher and Moody (1939) J Lab Clin Med, 1183-1189). Thus, the present patent application refers also to the use of all tautomers of 5-amino-2,3-dihydro-1.4-phthalazinedione and its pharmaceutically acceptable salts, hydrates and solvates.

Isomer is a generic term for molecules with the same chemical formula but a different chemical structure. They can be differentiated into constitutional (structural) isomers (wherein an exchange of atoms or of a functional group occurs) and stereoisomers. Stereoisomers can be subdivided into enantiomers (non-superimposable mirror images of the same molecule) and diastereomers (the same molecule with a different configuration at one or more stereocenters). Diastereomers can be subdivided into cis/trans isomers (referring to the relative orientation of functional groups within a molecule) and on the other hand conformers (rotation about formally single bonds) and rotamers (different rotational positioning about a single bond). An example for a constitutional isomer of 5-amino-2,3-dihydro-1,4-phthalazinedione is 6-amino-2,3-dihydrophthalazine-1,4-dione (isoluminol). Stereoisomers may occur in phthalazinedione derivatives. Thus, the present patent application refers also to the use of all isomers of 5-amino-2,3-dihydro-1,4-phthalazinedione, its derivatives and pharmaceutically acceptable salts, hydrates and solvates.

For some applications it may be desirable that Isotopically enriched forms of the compounds of the invention are used e.g., for diagnostic purposes. Thus, the present patent application refers also to such isotopically enriched forms of the compounds of the invention.

From a pharmacokinetic point of view or for a production rationale it may be preferable to use a prodrug as a dosage form. A prodrug is administered in a pharmacologically inactive form and is metabolically converted into the active form inside the body. This conversion may occur systemically or locally. Thus, the present patent application refers also to prodrugs of the compounds of the invention.

As used throughout the present application the term "5-amino-2,3-dihydro-1,4-phthalazinedione or one of its pharmaceutically acceptable salts, hydrates or solvates" shall encompass all the beforementioned molecular variants of 5-amino-2,3-dihydro-1,4-phthalazinedione i.e., 5-amino2,3-dihydro-1,4-phthalazinedione or one of its pharmaceutically acceptable salts, hydrates or solvates, crystalline polymorphs, tautomers or isotopically enriched forms thereof.

Unless otherwise stated, any technical or scientific term used in the present invention has the meaning that a man skilled in the relevant technical art will attribute to them.

The terms "composition" or "pharmaceutical composition" comprise at least one active ingredient in any pharmacologically acceptable defined dosage and dosage form together with at least one pharmaceutically acceptable excipient, as well as all agents that are generated from the ingredients as outlined below directly or indirectly as a combination, accumulation, complex or crystal, or as a consequence of other reactions or interactions, as well as optionally at least one further pharmaceutical drug, as listed below.

The term "excipient" is used in this application to describe any component of a pharmaceutical composition apart of the pharmaceutically active principle. The selection of suitable excipients depends on a variety of factors, such as the dosage form, the dosage, the desired solubility and the stability of the composition.

The terms "effect", "therapeutic effect", "action", "therapeutic action", "efficacy" and "effectiveness" with regard to the pharmaceutical combination of the disclosure or any other active substance mentioned in the description refers to causally occurring beneficial consequences in the organism to which said substance has been administered before.

According to the invention the terms "effective amount" and "therapeutically effective amount" refer to an amount of the substance of the invention that is sufficiently large to cause a desired beneficial effect in a subject in need of such a treatment.

The terms "treatment" and "therapy" comprise the administration of at least the substance of the invention, alone or In combination with at least one further pharmaceutical drug, independently of the chronological order of the administration. Such an administration is intended to substantially improve the disease course of a multiple sclerosis-type disease or a psoriasis-type disease by either completely curing the disease or by stopping or decelerating the increase of disabilities during the course of the disease-

The terms "prophylaxis" or "prophylactic treatment" comprise the administration of at least the substance of the invention, alone or in combination with at least one further pharmaceutical drug, independently of the chronological order of the administration, for preventing or suppressing the manifestation of symptoms attributed to a multiple sclerosis-type disease or a psoriasis-type disease. It refers particularly to medical conditions of a patient in which the manifestation of such symptoms is expected to occur in the far or near future with a reasonable probability.

The terms "subject" and "patient" comprise individuals suffering from disease symptoms or disabilities related to a multiple sclerosis-type disease or a psoriasis-type disease wherein said diagnosis is either approved or suspected. Individuals are mammals, in particular humans.

The pharmaceutical combination of 5-amino-2,3-dihydro-1,4-phthalazinedione or one of its pharmaceutically acceptable salts, hydrates or solvates and at least one fumaric acid ester or a pharmaceutically acceptable salt thereof can be used as a single therapy or can further be combined with at least one further active ingredient selected from a group comprising active ingredients used in disease-modifying therapies for multiple sclerosis-type diseases or psoriasis-type diseases, in symptomatic therapies of them and in the treatment of comorbidities.

The pharmaceutical combination of 5-amino-2,3-dihydro-1.4-phthalazinedione or one of its pharmaceutically acceptable salts, hydrates or solvates and at least one fumaric acid ester or a pharmaceutically acceptable salt thereof can be used simultaneously, separately or sequentially for treating or preventing disease symptoms. The at least two active agents may be provided in a single dosage form or as separate formulation, each formulation containing at least one of the two active agents. One or any of the active agents may be formulated as a bolus.

In particular, the present application discloses a pharmaceutical combination of 5-amino-2,3-dihydro-1,4-phthalazinedione or one of its pharmaceutically acceptable salts and at least one fumaric acid ester or a pharmaceutically acceptable salt thereof for use in the treatment of a multiple sclerosis-type disease or a psoriasis-type disease, wherein a previous treatment with at least one other pharmaceutically active agent was refractory.

The terms "medicine" or "medical" comprise human as well as veterinary medicine.

The term "organism" refers to a living being, especially a human or an animal, possessing a self-regulating immunological system.

The term "active agent" in this application refers to 5-amino-2,3-dihydro-1,4-phthaIazinedione or one of its pharmaceutically acceptable salts, hydrates or solvates or to at least one fumaric acid ester or a pharmaceutically salt thereof, if not stated otherwise. Moreover, this term can comprise further pharmaceutical agents, known from the state of the art.

The terms "composition" and "pharmaceutical composition" comprise a pharmaceutical combination of 5-amino-2,3-dihydro-1,4-phthaiazinedione or one of its pharmaceutically acceptable salts, hydrates or solvates and at least one fumaric acid ester or a pharmaceutically acceptable salt thereof in any pharmacologically suitable defined dose and dosage form together with at least one suitable excipient and carrier substance as well as all substances which are directly or indirectly generated as a combination, accumulation, complex formation or crystal of the aforementioned ingredients, or come into being as a result of other reactions or interactions.

The term "excipient" is used in this application to describe each component of
a pharmaceutical composition in addition to the active agent. The selection of a suitable excipient depends on factors such as dosage form and dose as well as the influence on the solubility and stability of the composition by the excipient itself.

The term "action" describes the inherent specific mode of action of the respective agent in the scope of the present application.

The terms "effect", "therapeutic effect", "action", "therapeutic action" regarding at least one active agent according to the invention refer to causally occurring beneficial consequences for the organism, to which the at least one active agent has been administered.

In terms of the application, "therapeutically effective dose" means that a sufficient dose of a pharmaceutical combination of 5-amino-2,3-dihydro-1,4-phthalazinedione or one of its pharmaceutically acceptable salts, hydrates or solvates and at least one fumaric acid ester or a pharmaceutically acceptable salt thereof is administered to a living being or to a patient in need of such a treatment.

The terms "joint administration", "combined administration" or "simultaneous administration" of at least one pharmaceutical agent according to the invention and/or of at least one pharmaceutical agent from the state of the art comprise the administration of the mentioned agents at the same time or at time points factually related close to each other, as well as administrations of said agents at different times within a coherent experiment. The chronological order of the administration of said agents is not limited by these terms. Those skilled in the art will have no difficulties to deduce the described administrations in respect to their chronological or local order from his knowledge and experience.

The term "living being" refers to every animal, especially vertebrate, including human. A "patient" in terms of the application is a living being who suffers from a definable and diagnosable disease, and to whom a suitable active agent can be administered.

The terms "prophylaxis", "treatment" and "therapy" comprise the administration of a pharmaceutical combination of 5-amino-2.3-dihydro-1,4-phthalazinedione or one of its pharmaceutically acceptable salts, hydrates or solvates and at least one fumaric acid ester or a pharmaceutically acceptable salt thereof to a living being, in order to prevent the development of a certain disease, to inhibit, and to alleviate the symptoms, or to initiate a healing process of the respective disease.

The pharmaceutical combination of 5-amino-2,3-dihydro-1,4-phthalazinedione or one of its pharmaceutically acceptable salts, hydrates or solvates and at least one fumaric acid ester or a pharmaceutically acceptable salt thereof can be applied in the prophylaxis or treatment of a multiple sclerosis-type disease or a psoriasis-type disease by any medically acceptable administration route to a patient in need thereof. Such medically acceptable administration routes can be e.g., intravenous, peroral, sublingual, topical, parenteral, intraperitoneal, intraarterial, intramuscular, dermal, transdermal, subcutaneous, intradermal, sublingual, intrathecal or intraventricular.

In particular, the present application refers to a pharmaceutical combination for use according to the disclosure, wherein said pharmaceutical combination is for use in the prophylaxis or treatment of a medical condition that is treated with at least one fumaric acid ester.

Preferred oral formulations for use in the prophylaxis or treatment of a multiple sclerosis-type disease or a psoriasis-type disease are capsules or tablets containing 5-amino-2,3-dihydro-1,4-phthalazinedione or one of its pharmaceutically acceptable salts, hydrates and solvates in an amount of 50 mg, 100 mg, 150 mg, 200 mg, 300 mg, 400 mg, 500 mg or 600 mg, preferably 100 mg, 150 mg, 200 mg, 300 mg or 400 mg, most preferably 300 mg and at least one fumaric acid ester or a pharmaceutically acceptable salt thereof.

In the scope of the present disclosure it is understood that any combinations of said dosages of 5-amino-2,3-dihydro-1,4-phthalazinedione or one of its pharmaceutically acceptable salts, hydrates and solvates with at least one fumaric acid ester or one of their pharmaceutically acceptable salts are disclosed.

In another aspect of the invention a pharmaceutical composition for use in the prophylaxis or treatment of a multiple sclerosis-type disease or a psoriasis-type disease is disclosed, wherein said pharmaceutical composition contains 5-amino-2,3-dihydro-1,4-phthalazinedione or one of its pharmaceutically acceptable salts, hydrates or solvates and at least one fumaric acid ester or a pharmaceutically acceptable salt thereof, a carrier and at least one pharmaceutically acceptable excipient.

The term "pharmaceutically acceptable excipient(s)" refers to natural or synthetic compounds that are added to a pharmaceutical formulation alongside the pharmaceutical active agent. They may help to bulk up the formulation, to enhance the desired pharmacokinetic properties or the stability of the formulation, as well as being beneficial in the manufacturing process. Advantageous classes of excipients according to the invention include carriers, binding agents, colorants, buffers, preservatives, antioxidants, coatings, sweeteners, thickening agents, pH-regulators, acidity regulators, acidifiers, solvents, isotonizing agents, disintegrants, glidants, lubricants, emulsifiers, solubilizing agents, stabilizers, diluents, anti-caking agents (anti-adherents), permeation enhancers, sorbents, foaming agents, anti-foaming agents, opacifiers, fatliquors, consistency enhancers, hydrotropes, aromatic and flavoring substances.

In general, one or more pharmaceutically acceptable carriers are added to a pharmaceutically active agent. Eligible are all carriers known in the art and combinations thereof. In solid dosage forms they can be for example plant and animal fats, waxes,. paraffins, starch, tragacanth, cellulose derivatives, polyethylene glycols, silicones, bentonites, silica, talcum, zinc oxide. For liquid dosage forms and emulsions suitable carriers are for example solvents, solubilizing agents, emulsifiers such as water, ethanol, isopropanol, ethyl carbonate, ethyl acetate, benzyl alcohol, benzyl benzoate, propylene glycol, 1,3-butyl glycol, cotton seed oil, peanut oil, olive oil, castor oil, sesame oil, glycerol fatty acid esters, polyethylglycols, fatty acid esters of sorbitan. Suspensions according to the invention may use carriers known in the art such as diluents (e.g., water, ethanol or propylene glycol), ethoxylated isostearyl alcohols, polyoxyethylene and polyoxyethylene sorbitan esters, microcrystalline cellulose, bentonites, agar agar, tragacanth.

The term binding agents refers to substances that bind powders or glue them together, rendering them cohesive through granule formation. They serve as a "glue" of the formulation. Binding agents increase the cohesive strength of the provided diluent or filler.

Suitable binding agents are for example starch from wheat, corn, rice or potato, gelatin, naturally occurring sugars such as glucose, sucrose or beta-lactose, sweeteners from corn, natural and synthetic gums such as acacia, tragacanth or ammonium calcium alginate, sodium alginate, carboxymethyl cellulose, sodium carboxymethyl cellulose, hydroxypropyl carboxymethyl cellulose, polyethylene glycol, polyvinyl pyrrolidone, magnesium aluminum silicate, waxes and others. The percentage of the binding agent in the composition can range from 1 - 30 % by weight, preferred 2 - 20 % by weight, more preferred 3 - 10 % by weight and most preferred 3 - 6 % by weight.

Colorants are excipients that bestow a colorization to the pharmaceutical formulation. These excipients can be food colorants. They can be adsorbed on a suitable adsorption means such as clay or aluminum oxide. A further advantage of a colorant is that it may visualize spilled aqueous solution on the nebulizer and/or the mouthpiece to facilitate cleaning. The amount of the colorant may vary between 0.01 and 10 % per weight of the pharmaceutical composition, preferred between 0.05 and 6 % per weight, more preferred between 0.1 and 4 % per weight, most preferred between 0.1 and 1 % per weight.

Suitable pharmaceutical colorants are for example curcumin, riboflavin, riboflavin-5'-phosphate, tartrazine, alkannin, quinolione yellow WS, Fast Yellow AB, riboflavin-5'-sodium phosphate, yellow 2G, Sunset yellow FCF, orange GGN, cochineal, carminic acid, citrus red 2, carmoisine, amaranth. Ponceau 4R, Ponceau SX, Ponceau 6R, erythrosine, red 2G, Allura red AC. Indathrene blue RS, Patent blue V, indigo carmine, Brilliant blue FCF, chlorophylls and chlorophyllins, copper complexes of chlorophylls and chlorophyllins, Green S, Fast Green FCF, Plain caramel, Caustic sulfite caramel, ammonia caramel, sulfite ammonia caramel, Black PN, Carbon black, vegetable carbon. Brown FK, Brown HT, alpha-carotene, beta-carotene, gamma-carotene, annatto, bixin, norbixin, paprika oleoresin, capsanthin, capsorubin, lycopene, beta-apo-8'-carotenal, ethyl ester of beta-apo-8'-carotenic acid, flavaxanthin, lutein, cryptoxanthin, rubixanthin, violaxanthin, rhodoxanthin, canthaxanthin, zeaxanthin, citranaxanthin, astaxanthin, betanin, anthocyanins, saffron, calcium carbonate, titanium dioxide, iron oxides, iron hydroxides, aluminum, silver, gold, pigment rubine, tannin, orcein, ferrous gluconate, ferrous lactate.

Moreover, buffer solutions are preferred for liquid formulations, in particular for pharmaceutical liquid formulations. The terms buffer, buffer system and buffer solution, in particular an aqueous buffer solution, refer to the capacity of the system to resist a pH change by the addition of an acid or a base, or by dilution with a solvent. Preferred buffer systems may be selected from the group comprising formate, lactate, benzoic acid, oxalate, fumarate, aniline, acetate buffer, citrate buffer, glutamate buffer, phosphate buffer, succinate, pyridine, phthalate, histidine, MES (2-(N-morpholino) ethanesulfonic acid), maleic acid, cacodylate (dimethyl arsenate), carbonic acid, ADA (N-(2-acetamido)imino diacetic acid, PIPES (4-piperazine-bis-ethenesutfonic acid), BIS-TRIS propane (1,3-bis[tris(hydroxymethyl)methylamino] propane), ethylene diamine, ACES (2-[(amiho-2-oxoethyl)amino]ethanesulfonic acid). imidazole, MOPS (3-(N-morpholino) propanesulfonic acid), diethyl malonic acid, TES (2-[tris(hydroxymethyl)methyl]aminoethanesulfonic acid), HEPES (N-2-hydroxyethylpiperazine-N'-2-ethanesulfonic acid), as well as other buffers with a pKₐ between 3.8 and 7.7.

Preferred are carbonic acid buffers such as acetate buffer and dicarboxylic acid buffers such as fumarate, tartrate and phthalate as well as tricarboxylic acid buffers such as citrate.

A further group of preferred buffers are inorganic buffers such as sulfate hydroxide, borate hydroxide, carbonate hydroxide, oxalate hydroxide, calcium hydroxide and phosphate buffers. Another group of preferred buffers are nitrogen-containing puffers such as imidazole, diethylene diamine and piperazine. Furthermore preferred are sulfonic acid buffers such as TES, HEPES, ACES, PIPES, [(2-hydroxy-1,1-bis-(hydroxymethyl)ethyl)amino]-1-propanesulfonic acid (TAPS), 4-(2-hydroxyethyl)piperazine-1-propanesulfonic acid (EEPS), MOPS and N,N-bis-(2-hydroxyethyl}-2-aminoethanesulfonic acid (BES). Another group of preferred buffers are glycine, glycyl-glycine, glycyl-glycyl-glycine, N,N-bis-(2-hydroxyethyl)glycine and N-[2-hydroxy-1,1-bis(hydroxymethyl)ethyl]glycine (tricine). Preferred are also amino acid buffers such as glycine, alanine, valine, leucine, isoleucine, serine, threonine, phenylalanine, tyrosine, tryptophan, lysine, arginine, histidine, aspartate, glutamate, asparagine, glutamine, cysteine, methionine, proline, 4-hydroxy proline, *N,N,N-*trimethyllysine, 3-methyl histidine, 5-hydroxy lysine, o-phosphoserine, gamma-carboxyglutamate, [epsilon]-*N*-acetyl lysine, [omega]-*N*-methyl arginine, citrulline, ornithine and their derivatives. Particularly preferred is KH₂PO₄ buffer.

Preservatives for liquid and/or solid dosage forms can be used on demand. They may be selected from the group comprising, but not limited to, sorbic acid, potassium sorbate, sodium sorbate, calcium sorbate, methyl paraben, ethyl paraben, methyl ethyl paraben, propyl paraben, benzoic acid, sodium benzoate, potassium benzoate, calcium benzoate, heptyl p-hydroxybenzoate, sodium methyl para-hydroxybenzoate, sodium ethyl para-hydroxybenzoate, sodium propyl para-hydroxybenzoate, benzyl alcohol, benzalkonium chloride, phenylethyl alcohols, cresols, cetylpyridinium chloride, chlorobutanol, thiomersal (sodium 2-(ethylmercurithio) benzoic acid), sulfur dioxide, sodium sulfite, sodium bisulfite, sodium metabisulfite, potassium metabisulfite, potassium sulfite, calcium sulfite, calcium hydrogen sulfite, potassium hydrogen sulfite, biphenyl, orthophenyl phenol, sodium orthophenyl phenol, thiabendazole, nisin, natamycin, formic acid, sodium formate, calcium formate, hexamine, formaldehyde, dimethyl dicarbonate, potassium nitrite, sodium nitrite, sodium nitrate, potassium nitrate, acetic acid, potassium acetate, sodium acetate, sodium diacetate, calcium acetate, ammonium acetate, dehydroacetic acid, sodium dehydroacetate, lactic acid, propionic acid, sodium propionate, calcium propionate, potassium propionate, boric acid, sodium tetraborate, carbon dioxide, malic acid, fumaric acid, lysozyme, copper-(ll)-sulfate, chlorine, chlorine dioxide and other suitable substances or compositions known to the person skilled in the art.

The addition of a sufficient amount of antioxidants is particularly preferable for liquid and topical dosage forms. Suitable examples for antioxidants include sodium metabisulfite, alpha-tocopherol, ascorbic acid, maleic acid, sodium ascorbate, ascorbyl palmitate, butylated hydroxyanisole, butylated hydroxytoluene, fumaric acid or propyl gallate. Preferred is the use of sodium metabisulfite, alpha-tocopherol and ascorbyl palmitate.

Tablets or pills are usually coated i.e., the coating constitutes the outer layer. This can be a film coating, a sugar coating with saccharides and a compression coating. Pharmaceutically acceptable varnishes or waxes, HPMC (hydroxypropylmethylcellulose), MC (methylcellulose) or HPC (hydroxypropylcellulose) can be used. Such a coating may help to disguise the taste, to ease the swallowing or the identification. Often plasticizers and pigments are included in the coating. Capsules normally have a gelatinous envelope that encloses the pharmaceutical combination of the disclosure. The specific composition and thickness of this gelatinous layer determines how fast absorption takes place after ingestion of the capsule. Of special interest are sustained release formulations, as known in the art.

Suitable sweeteners can be selected from the group comprising mannitol, glycerol, acesulfame potassium, aspartame, cyclamate, isomalt, isomaltitol, saccharin and its sodium, potassium and calcium salts, sucralose, alitame, thaumatin, glycyrrhizin, neohesperidine dihydrochalcone, steviol glycosides, neotame, aspartame-acesulfame salt, maltitol, maltitol syrup, lactitol, xylitol, erythritol.

Suitable thickening agents can be selected from the group comprising, but not limited to, polyvinyl pyrrolidone, methyl cellulose, hydroxypropyl methyl cellulose, hydroxypropyl cellulose, dextrins, polydextrose, modified starch, alkaline modified starch, bleached starch, oxidized starch, enzyme-treated starch, monostarch phosphate, distarch phosphate esterified with sodium trimetaphosphate or phosphorus oxychloride, phosphate distarch phosphate, acetylated distarch phosphate, starch acetate esterified with acetic anhydride, starch acetate esterified with vinyl acetate, acetylated distarch adipate, acetylated distarch glycerol, distarch glycerin, hydroxypropyl starch, hydroxy propyl distarch glycerin, hydroxypropyl distarch phosphate, hydroxypropyl distarch glycerol, starch sodium octenyl succinate, acetylated oxidized starch, hydroxyethyl cellulose.

Suitable pH-regulators for liquid dosage forms are e.g., sodium hydroxide, hydrochloric acid, buffer substances such as sodium dihydrogen phosphate or disodium hydrogenphosphate.

Suitable acidity regulators can be selected from the group comprising acetic acid, potassium acetate, sodium acetate, sodium diacetate, calcium acetate, carbon dioxide, malic acid, fumaric acid, sodium lactate, potassium lactate, calcium lactate, ammonium lactate, magnesium lactate, citric acid, mono-, di-, trisodium citrate, mono-, di-, tripotassium citrate, mono-, di-, tricalclum citrate, tartaric acid, mono-, disodium tartrate, mono-, dipotassium tartrate, sodium potassium tartrate, ortho-phosphoric acid, lecithin citrate, magnesium citrate, ammonium malate, sodium malate. sodium hydrogen malate, calcium malate, calcium hydrogen malate, adipic acid, sodium adipate. potassium adipate, ammonium adipate, succinic acid, sodium fumarate, potassium fumarate, calcium fumarate, ammonium fumarate, 1,4-heptonolactone, triammonium citrate, ammonium ferric citrate, calcium glycerophosphate, isopropyl citrate, potassium carbonate, potassium bicarbonate, ammonium carbonate, ammonium bicarbonate, magnesium carbonate, magnesium bicarbonate, ferrous carbonate, ammonium sulfate, aluminum potassium sulfate, aluminum ammonium sulfate, sodium hydroxide, potassium hydroxide, ammonium hydroxide. magnesium hydroxide, gluconic acid.

Acidifiers use to be inorganic chemicals that either produce or become acid. Suitable examples are: Ammonium chloride, calcium chloride.

Suitable solvents may be selected from the group comprising, but not limited to, water, carbonated water, water for injection, water with isotonizing agents, saline, isotonic saline, alcohols, particularly ethyl and n-butyl alcohol, and mixtures thereof.

Suitable isotonizing agents are for example pharmaceutically acceptable salts, in particular sodium chloride and potassium chloride, sugars such as glucose or lactose, sugar alcohols such as mannitol and sorbitol, citrate, phosphate, borate and mixtures thereof.

Suitable disintegrants can be selected from the group comprising starch, cold water-soluble starches such as carboxymethyl starch, cellulose derivatives such as methyl cellulose and sodium carboxymethyl cellulose, microcrystalline cellulose and cross-linked microcrystalline celluloses such as croscarmellose sodium, natural and synthetic gums such as guar, agar, karaya (Indian tragacanth), locust bean gum, tragacanth, clays such as bentonite, xanthan gum. alginates such as alginic acid and sodium alginate, foaming compositions i.a, Moisture expansion is supported by for example starch, cellulose derivatives, alginates, polysaccharides, dextrans, cross-linked polyvinyl pyrrolidone. The amount of the disintegrant in the composition may vary between 1 and 40% per weight, preferred between 3 and 20% per weight, most preferred between 5 and 10% per weight.

Glidants are materials that prevent a baking of the respective supplements and improve the flow characteristics of granulations so that the flow is smooth and constant. Suitable glidants comprise silicon dioxide, magnesium stearate, sodium stearate, starch and talcum. The amount of the glidant in the composition may vary between 0.01 and 10% per weight, preferred between 0.1 and 7% per weight, more preferred between 0.2 and 5% per weight, most preferred between 0.5 and 2% per weight.

The term "lubricants" refers to substances that are added to the dosage form for facilitating tablets, granulates etc. to be released from the press mold or the outlet nozzle. They diminish friction or abrasion. Lubricants are usually added shortly before pressing, as they should be present on the surface of the granules and between them and the parts of the press mold. The amount of the lubricant in the composition may vary between 0.05 and 15% per weight, preferred between 0,2 and 5% per weight, more preferred between 0.3 and 3% per weight, most preferred between 0.3 and 1.5% per weight. Suitable lubricants are i.a. sodium oleate, metal stearates such as sodium stearate, calcium stearate, potassium stearate and magnesium stearate, stearic acid, sodium benzoate, sodium acetate, sodium chloride, boric acid, waxes having a high melting point, polyethylene glycol.

Emulsifiers can be selected for example from the following anionic and non-ionic emulsifiers: Anionic emulsifier waxes, cetyl alcohol, cetylstearyl alcohol, stearic acid, oleic acid, polyoxyethylene polyoxypropylene block polymers, addition products of 2 to 60 mol ethylene oxide to castor oil and/or hardened castor oil, wool wax oil (lanolin), sorbitan esters, polyoxyethylene alkyl esters, polyoxyethylene sorbitan fatty acid esters, polyoxyethene sorbitan monolaurate. polyoxyethene sorbitan monooleate, polyoxyethene sorbitan monopalmitate, polyoxyethene sorbitan monostearate, polyoxyethene sorbitan tristearate, polyoxyethene stearate, polyvinyl alcohol, metatartaric acid, calcium tartrate, alginic acid, sodium alginate, potassium alginate, ammonium alginate, calcium alginate, prppane-1,2-diol alginate, carrageenan, processed Eucheuma seaweed, locust bean gum, tragacanth, acacia gum, karaya gum, gellan gum, gum ghatti, glucomannane, pectin, amidated pectin, ammonium phosphatides, brominated vegetable oil, sucrose acetate isobutyrate, glycerol esters of wood rosins, disodium phosphate, trisodium diphosphate, tetrasodium diphosphate, dicalcium diphosphate, calcium dihydrogen diphosphate, sodium triphosphate, pentapotassium triphosphate, sodium polyphosphates, sodium calcium polyphosphate, calcium polyphosphates, ammonium polyphosphate, beta-cyclodextrin, powdered cellulose, methyl cellulose, ethyl cellulose, hydroxypropyl cellulose, hydroxypropyl methylcellulose, ethyl methyl cellulose, carboxymethyl cellulose, sodium carboxymethyl cellulose, ethyl hydroxyethyl cellulose, croscarmellose, enzymically hydrolyzed carboxymethyl cellulose, mono- and diglycerides of fatty acids, glyceryl monostearate, glyceryl distearate, acetic acid esters of mono- and diglycerides of fatty acids, lactic acid esters of mono- and diglycerides of fatty acids, citric acid esters of mono- and diglycerides of fatty acids, tartaric acid esters of mono- and diglycerides of fatty acids, mono- and diacetyl tartaric acid esters of mono- and diglycerides of fatty acids, mixed acetic and tartaric acid esters of mono- and diglycerides of fatty acids, succinylated monoglycerides, sucrose esters of fatty acids, sucroglycerides, polyglycerol esters of fatty acids, polyglycerol polyricinoleate, propane-1,2-diol esters of fatty acids, propylene glycol esters of fatty acids, lactylated fatty acid esters of glycerol and propane-1, thermally oxidized soy bean oil interacted with mono- and diglycerides of fatty acids, dioctyl sodium sulphosuccinate, sodium stearoyl-2-lactylate. calcium stearoyl-2-lactylate, stearyl tartrate, stearyl citrate, sodium stearoyl fumarate, calcium stearoyl fumarate, stearyl tartrate, stearyl citrate, sodium stearoyl fumarate, calcium stearoyl fumarate, sodium laurylsulfate, ethoxylated mono- and diglycerides, methyl glucoside-coconut oil ester, sorbitan monostearate, sorbitan tristearate, sorbitan monolaurate, sorbitan monooleate, sorbitan monopalmitate, sorbitan trioleate, calcium sodium polyphosphate, calcium polyphosphate, ammonium polyphosphate, cholic acid, choline salts, distarch glycerol, starch sodium octenyl succinate, acetylated oxidized starch. Preferred are glycerin monooleate, stearic acid, phospholipids such as lecithin.

Suitable as surface-active solubilizing agents (solubilizers) are for example diethylene glycol monoethyl ester, polyethyl propylene glycol co-polymers, cyclodextrins such as α- and β-cyclodextrin, glyceryl monostearates such as Solutol HS 15 (Macrogol-15-hydroxystearate from BASF, PEG 660-15 hydroxystearates), sorbitan esters, polyoxyethylene glycol, polyoxyethylene sorbitanic acid esters, polyoxyethylene sorbitan monooleate, polyoxyethylene oxystearic acid triglyceride, polyvinyl alcohol, sodium dodecyl sulfate, (anionic) glyceryl monooleates.

Stabilizers are substances that can be added to prevent unwanted changes. Though stabilizers are not real emulsifiers they may also contribute to the stability of emulsions.

Suitable examples for stabilizers are oxystearin, xanthan gum, agar, oat gum, guar gum, tara gum, polyoxyethene stearate, aspartame-acesulfame salt, amylase, proteases, papain, bromelain, ficin, invertase, polydextrose, polyvinyl pyrrolidone, polyvinyl polypyrrolidone, triethyl citrate, maltitol, maltitol syrup.

Diluents or fillers are inactive substances added to drugs for handling minimal amounts of active agents. Examples for suitable diluents are water, mannitol, pre-gelatinized starch,` starch, microcrystalline cellulose, powdered cellulose, silicified microcrystalline cellulose, dibasic calcium phosphate dihydrate, calcium phosphate, calcium carbonate, hydroxypropyl cellulose, hydroxyethyl cellulose, hydroxypropyl methylcellulose, polyethylene glycol, xanthan gum, gum arabic or any combination thereof.

Anti-caking agents (anti-adherents) can be added to a supplement or a composition of supplements for preventing the formation of lumps and for easing packaging, transport, release from the at least one chamber of the dispensing cap and consumption. Suitable examples include tricalcium phosphate, powdered cellulose, magnesium stearate. sodium bicarbonate, sodium ferrocyanide, potassium ferrocyanide, calcium ferrocyanide, bone phosphate, sodium silicate, silicon dioxide, calcium silicate, magnesium trisilicate, talcum powder, sodium aluminosilicate, potassium aluminum silicate, calcium aluminosilicate, bentonite, aluminum silicate, stearic acid, polydimethyl siloxane.

Sorbents are materials that soak up oil from the water. Suitable examples include natural sorbents such as peat moss, sawdust, feathers, and anything else natural that contains carbon and synthetic sorbents such as polyethylene and nylon. Sorbents are used for tablet/capsule moisture-proofing by limited fluid sorbing (taking up of a liquid or a gas either by adsorption or by adsorption) in a dry state.

In some galenic formulations it may be desirable that a liquid oral dosage form generates some foam on being dissolved. Such an effect can be supported through the addition of a foaming agent that reduces the surface tension of the liquid, thus facilitating the formation of bubbles, or it increases its colloidal stability by inhibiting coalescence of bubbles. Alternatively, it may stabilize foam. Suitable examples include mineral oil, quillaia extract, triethyl citrate, sodium lauryl ether sulfate, sodium lauryl sulfate, ammonium lauryl sulfate.

Alternatively, some liquid oral dosage forms may appear slightly foamy upon preparation. Though this does not interfere with the desired application it may affect patient compliance in case of a medication or the commercial success in case of dietary supplements. Therefore, it may be desirable to add a pharmaceutically acceptable anti-foaming agent (defoamer). Examples are polydimethylsiloxane or silicone oil in dietary supplements or simethicone in pharmaceuticals.

Opacifiers are substances that render the liquid dosage for, opaque, if desired. They must have a refractive index substantially different from the solvent, in most cases here water. At the same time, they should be inert to the other components of the composition. Suitable examples include titanium dioxide, talc, calcium carbonate, behenic acid, cetyl alcohol, or mixtures thereof.

Suitable fatliquors are e.g., oleic acid decyl ester, hydrated castor oil, light mineral oil, mineral oil, polyethylene glycol, sodium laurylsulfate.

Consistency enhancers are e.g., cetyl alcohol, cetyl ester wax, hydrated castor oil, microcrystalline waxes, non-ionic emulsifier waxes, beeswax, paraffin or stearyl alcohol.

Suitable hydrotropes are alcohols such as ethanol, isopropyl alcohol or polyols such as glycerin.

Suitable aromatic and flavoring substances comprise above all essential oils that can be used for this purpose. In general, this term refers to volatile extracts from plants or parts of plants with the respective characteristic smell. They can be extracted from plants or parts of plants by steam distillation.

Suitable examples are: Essential oils, respectively aromatic substances from achillea, sage, cedar, clove, chamomile, anise, aniseed, star anise, thyme, tea tree, peppermint, mint oil, menthol, cineol, borneol, zingerol, eucalyptus, mango, figs, lavender oil, chamomile blossoms, pine needle, cypress, orange, rose, rosewood, plum, currant, cherry, birch leaves, cinnamon, lime, grapefruit, tangerine, juniper, valerian, lemon, lemon balm, lemon grass, palmarosa, cranberry, pomegranate, rosemary, ginger, pineapple, guava, echinacea, ivy leave extract, blueberry, kaki, melon, alpha- or beta-pinene, alpha-pinene oxide, alpha-campholenic aldehyde, alpha-citronellol, alpha-isoamyl-cinnamic, alpha-cinnamic terpinene, alpha-terpineol, alpha-terpinene, aldehyde C₁₆, alpha-phellandrene, amyl cinnamic aldehyde, amyl salicylate, anisic aldehyde, basil, anethole, bay, benzyl acetate, benzyl alcohol, bergamot, bitter orange peel, black pepper, calamus, camphor, cananga oil, cardamom, carnation, carvacrol, carveol, cassia, castor, cedarwood, cinnamaldehyde, cinnamic alcohol, cis-pinane, citral, citronella, citronellal, citronellol dextro, citronellol, citronellyl acetate; citronellyl nitrile, citrus unshiu, clary sage, clove bud, coriander, corn, cotton seed, d-dihydrocarvone, decyl aldehyde, diethyl phthalate. dihydroanethole, dihydrocarveol, dihydrolinalool, dihydromyrcene, dihydromyrcenol, dihydromyrcenyl acetate; dihydroterpineol, dimethyl salicylate, dimethyloctanal, dimethyloctanol, dimethyloctanyl acetate, diphenyl oxide, dipropylene glycol, d-limonene, d-pulegone, estragole, ethyl vanillin, eucalyptol; eucalyptus citriodora, eucalyptus globulus, eugenol, evening primrose, fenchol, fennel, ferniol. fish, florazon, galaxolide, geraniol, geranium, geranyl acetate, geranyl nitrile. guaiacol, guaiacwood, gurjun balsam, heliotropin, herbanate, hiba, hydroxycitronellal, i-carvone, i-methyl acetate, ionone, isobutyl quinoleine, isobornyl acetate, isobornyl methylether, isoeugenol, isolongifolene, jasmine, lavender, limonene. linallol oxide, linallol, linalool, linalyl acetate, linseed, litsea cubeba, I-methyl acetate, longifolene, mandarin, mentha, menthane hydroperoxide, menthol crystals, menthol laevo, menthone laevo, methyl anthranilate, methyl cedryl ketone, methyl chavicol, methyl hexyl ether, methyl ionone, methyl salicylate, mineral, mint, musk ambrette, musk ketone, musk xylol, myrcene, nerol. neryl acetate, nonyl aldehyde, nutmeg, orris root, para-cymene, parahydroxy phenyl butanone crystals, patchouli, p-cymene, pennyroyal oil, pepper, perillaldehyde, petitgrain, phenyl ethyl alcohol, phenyl ethyl propionate, phenyl ethyl-2methylbutyrate, pimento berry, pimento leaf, pinane hydroperoxide, pinanol, pine ester, pine, pinene, piperonal, piperonyl acetate, piperonyl alcohol, plinol, plinyl acetate, pseudo ionone, rhodinol, rhodinyl acetate, rosalin, ryu, sandalwood, sandenol, sassafras, sesame, soybean, spearmint, spice, spike lavender, spirantol, starflower, tea seed, terpenoid, terpineol, terpinolene, terpinyl acetate, tert-butylcyclohexyl acetate, tetrahydrolinalool, tetrahydrolinalyl acetate, tetrahydromyrcenol, thulasi, thymol, tomato, trans-2-hexenol, trans-anethole, turmeric, turpentine, vanillin, vetiver, vitalizair, white cedar, white grapefruit, wintergreen etc. or mixtures thereof, as well as mixtures of menthol, peppermint and star anise oil or menthol and cherry flavor.

These aromatic or flavoring substances can be included in the range of 0.0001 to 10 % per weight (particularly in a composition), preferred 0.001 to 6% per weight, more preferred 0.001 to 4% per weight, most preferred 0.01 to 1% per weight, with regard to the total composition. Application- or single case-related it may be advantageous to use differing quantities.

According to the invention all the beforementioned excipients and classes of excipients can be used without limitation alone or in any conceivable combination thereof, as long as the inventive use is not thwarted, toxic actions may occur, or respective national legislations are infracted.

In another aspect of the invention the present application relates to a pharmaceutical combination of 5-amino-2,3-dihydro-1,4-phthalazinedione or one of its pharmaceutically acceptable salts, hydrates or solvates and at least one fumaric acid ester or a pharmaceutically acceptable salt thereof or a pharmaceutical composition according to the invention for use in a formulation for oral administration in the prophylaxis or treatment of a multiple sclerosis-type disease or a psoriasis-type disease.

Pharmaceutical formulations suitable for oral dosage forms of a pharmaceutical combination of 5-amino-2,3-dihydro-1,4-phthalazinedione or one of its pharmaceutically acceptable salts, hydrates or solvates and at least one fumaric ester or a pharmaceutically acceptable salt thereof may be administered as separate units such as tablets, soft gelatin capsules, hard gelatin capsules, sugar-coated tablets or pills; powders or granulates; juices, syrups, drops, teas, solutions or suspensions in aqueous or non-aqueous liquids; edible foams or mousses; or in oil-in-water or water-in-oil in emulsions.

In oral dosage forms such as tablets or capsules the active agent can thus be combined with a non-toxic and pharmaceutically acceptable inert carrier such as ethanol, glycerol or water. Powders are produced by grinding the compound to a suitably tiny particle size and mixing them with a pharmaceutical carrier in a similar manner e.g., an edible carbohydrate such as starch or mannitol. A flavor, preservative, dispersant or colorant can also be present.

Tablets are formulated by producing, granulating or dry-pressing a powder mixture, adding a lubricant and a disintegrants and pressing the mixture to a tablet. A powder mixture is produced by mixing a suitably ground compound with a diluent or a base as described before, and If applicable, with a binding agent such as carboxymethyl cellulose, an alginate, gelatin or polyvinyl pyrrolidone, a dissolution retardant, such as, for example, paraffin, an absorption accelerator, such as, for example, a quaternary salt, and/or an absorbent, such as. for example, bentonite, kaolin or dicalcium phosphate. The powder mixture can be granulated by wetting it with a binder, such as, for example, syrup, starch paste, acacia mucilage or solutions of cellulose or polymer materials and pressing it through a sieve. As an alternative to granulation, the powder mixture can be run through a tableting machine, giving lumps of non-uniform shape which are broken up to form granules. The granules can be lubricated by addition of stearic acid, a stearate salt, talc or mineral oil in order to prevent sticking to the tablet casting mold. The lubricated mixture is then pressed to give tablets. The compounds according to the invention can also be combined with a free-flowing inert excipient and then pressed directly to give tablets without carrying out the granulation or dry-pressing steps.

In another aspect of the invention a pharmaceutical combination of 5-amino-2,3-dihydro-1,4-phthalazinedione or one of its pharmaceutically acceptable salts, hydrates or solvates and at least one fumaric ester or a pharmaceutically acceptable salt thereof is provided in hard gelatin capsules. They are fabricated by producing a powder mixture as described before and filling it into shaped gelatin covers. Glidants and lubricants such as highly dispersed silica, talcum, magnesium stearate, calcium stearate or polyethylene glycol can be added to the powder mixture as solids. A disintegrant or solubilizer such as agar agar, calcium carbonate or sodium carbonate can be added likewise for improve the availability of the medication after intake of the capsule. Additionally, suitable binding agents and/or colorants can be added to the mixture, if desirable or necessary.

In another aspect of the invention a pharmaceutical combination of 5-amino-2,3-dihydro-1,4-phthalazinedione or one of its pharmaceutically acceptable salts, hydrates or solvates and at least one fumaric ester or a pharmaceutically acceptable salt thereof is included in soft gelatin capsules (SGC), SGC are dissolved on their passage through the gastrointestinal tract. They consist mainly of gelatin enriched with variable amounts of plasticizers such as glycerol or sorbitan. The release rate depends on the specific formulation of the SGC carrier material. They are also suitable for a sustained release of the active agent. SGC are particularly useful for the administration of poorly water-soluble active agents.

In another aspect of the invention a pharmaceutical combination of 5-amino-2,3-dihydro-1,4-phthalazinedione or one of its pharmaceutically acceptable salts, hydrates or solvates and at least one fumaric ester or a pharmaceutically acceptable salt thereof is included in chewable tablets or hard caramels. Herein the substance is integrated into the matrix of the tablets or caramels.

In yet another aspect of the invention a pharmaceutical combination of 5-amino-2,3-dihydro-1,4-phthalazinedione or one of its pharmaceutically acceptable salts, hydrates or solvates and at least one fumaric acid ester or a pharmaceutically acceptable salt thereof or a pharmaceutical composition according to the invention for use in the prophylaxis or treatment of a multiple sclerosis-type disease or a psoriasis-type disease, wherein said pharmaceutical combination or pharmaceutical composition is applied in form of liposomes, micelles, multilamellar vesicles or a cyclodextrin complex.

In yet another aspect of the invention the present application relates to a pharmaceutical combination of 5-amino-2.3-dihydro-1,4-phthalazinedione or one of its pharmaceutically acceptable salts, hydrates or solvates and at least one fumaric acid ester or a pharmaceutically acceptable salt thereof or a pharmaceutical composition according to the invention for use in the prophylaxis or treatment of a multiple sclerosis-type disease or a psoriasis-type disease in a formulation for sublingual tablets.

In yet another aspect of the invention the present application relates to a pharmaceutical combination of 5-amino-2,3-dihydro-1,4-phthalazinedione or one of its pharmaceutically acceptable salts, hydrates or solvates and at least one fumaric acid ester or a pharmaceutically acceptable salt thereof or a pharmaceutical composition according to the invention for use in the prophylaxis or treatment of a multiple sclerosis-type disease or a psoriasis-type disease in a liquid dosage form.

The present application discloses also the parenteral administration of a pharmaceutical combination of 5-amino-2,3-dihydro-1,4-phthalazinedione or one of its pharmaceutically acceptable salts, hydrates or solvates and at least one fumaric acid ester or a pharmaceutically acceptable salt thereof or a pharmaceutical composition according to the invention in the prophylaxis or treatment of a multiple sclerosis-type disease or a psoriasis-type disease in the form of intravenous injection, intraarterial injection or intraperitoneal injection.

These liquid dosage forms comprise solutions, suspensions and emulsions. Examples are water and water/propylene glycol solutions for parenteral injections, or the addition of a sweetener or opacifier for oral solutions, suspensions and emulsions.

These liquid dosage forms can be stored in vials. IV bags, ampoules, cartridges, and prefilled syringes. Suitable excipients include solubilizers, stabilizers, buffers, tonicity modifiers, bulking agents, viscosity enhancers/reducers, surfactants, chelating agents, and adjuvants.

In yet another aspect of the invention a pharmaceutical combination of 5-amino-2,3-dihydro-1,4-phthalazinedione or one of its pharmaceutically acceptable salts, hydrates or solvates and at least one fumaric acid ester or a pharmaceutically acceptable salt thereof or a pharmaceutical composition according to the invention for use in the prophylaxis or treatment of a multiple sclerosis-type disease or a psoriasis-type disease, wherein said pharmaceutical combination or pharmaceutical composition is formulated as a lyophilizate. A lyophilizate can be reconstituted with water for injection or physiological saline or a water/ethanol solution and then be administered by injection.

Typical application forms for intravenous injections include infusion pumps, hypodermic needles, drip chambers, peripheral cannulas (peripheral venous catheters) and pressure bags.

In general, an aqueous solution or a physiological saline solution is preferred. In case of a poorly soluble pharmaceutical agent according to the invention also ethanol or ethanol/water mixtures can be used.

Another suitable liquid dosage form are drops.

A gel is a colloid in which the solid disperse phase forms a network in combination with that of the fluid continuous phase, resulting in a viscous semirigid sol. Gel properties range from soft and weak to hard and tough. They are defined as a substantially dilute cross-linked system, which exhibits no flow in the steady-state. By weight, gels are mostly liquid, yet they behave like solids due to a three-dimensional cross-linked network within the liquid. It is the crosslinking within the fluid that gives a gel its consistency and contributes to the adhesive stick. Gels are a dispersion of molecules of a liquid within a solid medium.

A hydrogel is a network of polymer chains that are hydrophilic, sometimes found as
a colloidal gel in which water is the dispersion medium. From the hydrophilic polymer chains being held together by cross-links results a three-dimensional solid, Because of the inherent cross-links, the structural integrity of the hydrogel network does not dissolve from the high concentration of water. Hydrogels are highly absorbent (they can contain over 90% water) natural or synthetic polymeric networks. Hydrogels also possess a degree of flexibility very similar to natural tissue, due to their significant water content. In medicine, hydrogels can encapsulate chemical systems which upon stimulation by external factors such as a change of pH may cause specific pharmaceutically active agent(s) to be liberated to the environment, in most cases by a gel-sol transition to the liquid state.

Suitable gel formers can be selected from the group comprising, but not limited to, agar, algin, alginic acid, bentonite, carbomer, carrageenan, hectorite, hydroxyethyl cellulose, hydroxypropyl cellulose, polyvinyl alcohol, polyvinyl pyrrolidone, sodium carbomer.

Sublingual drug delivery can be an alternative when compared to oral drug delivery as sublingually administered dosage forms bypass hepatic metabolism. A rapid onset of pharmacological effect is often desired for some drugs, especially those used In the treatment of acute disorders. Sublingual tablets disintegrate rapidly, and the small amount of saliva present is usually sufficient for achieving disintegration of the dosage form coupled with better dissolution and increased bioavailability.

The drug must be lipophilic enough to be able to partition through the lipid bilayer, but not so lipophilic such that once it is in the lipid bilayer, it will not partition out again. According to the diffusive model of absorption, the flux across the lipid bilayer is directly proportional to the concentration gradient. Therefore, lower salivary solubility results in lower absorption rates and vice versa. In general, a drug which has been formulated for sublingual should ideally have a molecular weight of less than 500 to facilitate its diffusion. The oral cavity has a narrow pH range which lies between 5.0 to 7.0. The inclusion of a suitable buffer during the formulation of an ionizable drug makes it possible to control the pH of aqueous saliva.

For avoiding a possibly unpleasant taste or smell of the drug taste masking is needed. Sweeteners, flavors, and other taste-masking agents are essential components. Sugar-based excipients quickly dissolve in saliva and produce endothermic heat of dissolution. They create a pleasant feeling in the mouth and are most suitable for sublingual tablets along with other flavors.

Typical techniques for manufacturing sublingual tablets include direct compression, compression molding, freeze drying and hot melt extrusion (Khan et al. (2017) J Pharmaceut Res 16: 257-267).

For topical applications containing a combination of at least one fumaric acid ester or one of its pharmaceutically acceptable salts and 5-amino-2,3-dihydro-1,4-phthalazinedione or one of its pharmaceutically acceptable salts, hydrates or solvates, respectively a pharmaceutical composition according to the disclosure for use according to the invention creams, emulsions, lotions, gels, hydrogels, pastes, powders, ointments, liniment, films, liposomes, dermal patches, transdermal patches, transdermal sprays or suspensions are suitable.

Permeation enhancers are often used in topical dosage forms. Suitable permeation enhancers comprise all pharmaceutically acceptable permeation enhancers known in the art, such as, without being limiting, azones such as laurocapran, 1-dodecylazacycloheptan-2-one; sulfoxides such as dimethyl sulfoxide, DMAC, DMF; pyrrolidones such as 2-pyrrofidone, N-methyl-2-pyrrolidone; alcohols such as ethanol, 1,2-propandiol or decanol; glycols such as propylene glycol, diethylene glycol, tetraethylene glycol; fatty acids such as oleic acid, lauric acid, sodium lauryl sulfate, myristic acid, isopropyl myristic acid, capric acid; nonic surfactants such as polyoxyethylene-2-oleyl ether, polyoxyethylene-2-stearyl ether; terpenes; terpenoids; oxazolidinones; urea; ceramide analogs, azone analogs, menthol derivatives, etherified derivatives, esterified derivatives, transkarbams, carbamate salts, TXA derivatives, DDAIP (dodecyl 2-(dimethylamino) propanoate), DDAK, natural essential oils (all of them listed in Chen et al. (2014) Asian J. Pharm. Sc. 9, 51-64): citric acid esters such as triethyl citrate; hydrophobin polypeptides; alpha-bisabolol; dimethyl isosorbide (Arlasolve^{®} DMI); ethoxydiglycol. Preferred is 1,2-propandiol.

Typical examples for preservatives suitable for topical applications are e.g. benzyl benzoate, benzoic acid, benzyl alcohol, benzalkonium chloride, N-cetyl-N-N-trimethylammonium bromide (Cetrimid, Merck), chlorhexidine, chlorobutanol, chlorocresol, imidurea, parabens such as methyl, ethyl, propyl or butyl paraben, sodium methylparaben, sodium propylparaben, potassium sorbate, sodium benzoate, sodium propionate, phenol, phenoxyethanol, phenylethyl alcohol, phenylmercuriacetate, phenylmercuriborate, phenylmercurinitrate, sorbic acid or Thiomersal (sodium methylmercurithiosalicylate). Preferred are methylparaben, propylparaben as well as sodium methylparaben and sodium propylparaben.

The addition of antioxidants is particularly preferable in topical dosage forms. Suitable examples for antioxidants include sodium metabisulfite, alpha-tocopherol, ascorbic acid, maleic acid, sodium ascorbate, ascorbyl palmitate, butylated hydroxyanisole, butylated hydroxytoluene, fumaric acid or propyl gallate. Preferred is the use of sodium metabisulfite.

Suitable pH-regulators for topical dosage forms are e.g., sodium hydroxide, hydrochloric acid, buffer substances such as sodium dihydrogen phosphate or disodium hydrogen phosphate.

Cream preparations may also contain other excipients and additives, such as fatliquors, solvents, consistency enhancers or hydrotropes for improving the flow characteristics. Herein single as well as several substances from the same group of additives or excipients may be present in the mixture.

Surprisingly, it can be shown that the concomitant administration of a pharmaceutical combination according to the invention not only shows a general supraadditive effect, but also shows this effect over the whole great range of fixed ratios tested.

The term "ratio" or "fixed ratio" hereby refers to any kind of ratio between two components independent of the units used. Such a ratio is valid for weight, weight %, concentration specifications and any other feasible unit in the field of pharmaceuticals. Hence, for example a ratio of the at least one fumaric acid ester or a pharmaceutically acceptable salt thereof to 5-amino-2.3-dihydro-1,4-phthalazinedione or one of its pharmacologically acceptable salts of 1:10 could for example be implemented as 1mg:10mg, 1mg/kg:10mg/kg, 1mM/10mM or 1%:10% or in any other unit, as long as the ratio itself is 1:10.

The present patent application also refers to a pharmaceutical combination comprising of 5-amino-2,3-dihydro-1,4-phthalazinedione or one of its pharmaceutically acceptable salts, hydrates or solvates and at least one fumaric acid ester or a pharmaceutically acceptable salt thereof or a pharmaceutical composition according to the invention for use in the prophylaxis or treatment of a multiple sclerosis-type disease or a psoriasis-type disease, wherein the efficacy of the prophylaxis or treatment is significantly improved compared to the respective treatment with said at least one fumaric acid ester alone.

Further, the pharmaceutical combination according to the disclosure can thus be used in the prophylaxis or treatment of a multiple sclerosis-type disease or a psoriasis-type disease, wherein the components can be used in any ratio.

However, to receive the best effects the medical indication to be treated, the potency of the pharmaceutical combination according to the disclosure, the application form and feasibility of the ratio should be considered from a practical point of view, the latter particularly for maintaining patient compliance.

In the following some possible combinations and respective ratios are provided, however, the pharmaceutical combinations according to the invention are not limited to these examples.

Depending on application form, indication to be treated and patients personal risk situation the at least one fumaric acid ester is administered for example as capsules (Tecfidera^{®}), as gastric acid-resistant tablets (Fumaderm^{®}) or as tablets (Skilarence^{®}).

Fumaric acid esters need to be administered cautiously, starting with a low dose for the first week and then continuously increasing the dosage.

Typical dosages of Tecfidera^{®} (dimethyl fumarate) in the treatment of relapsing-remitting multiple sclerosis start with a single dose of 120 mg every 12 hours (q12hr) i.e., 240 mg per day and are increased to 240 mg q12hr i.e., 480 mg per day.

Typical dosages of Fumaderm^{®}(a composition of 120 mg dimethyl fumarate, 87 mg ethyl hydrogen fumarate (synonym: monomethyl fumarate) calcium salt, 5 mg ethyl hydrogen fumarate magnesium salt and 3 mg ethyl hydrogen fumarate zinc salt per tablet) in the treatment of moderate-to-severe psoriasis start with 215 mg q24hr. This dosage is continuously increased to two tablets à 215 mg q8hr i.e., 1290 mg per day.

Typical dosages of Skilarence^{®}(dimethyl fumarate) in the treatment of moderate-to-severe psoriasis start with 30 mg q24hr. This dosage is continuously increased over several weeks to two tablets à 120 mg q8hr i.e., 720 mg per day.

5-amino-2,3-dihydro-1,4-phthalazinedione sodium salt has been shown to be very safe, however, due to compliance reasons dosages should not exceed 10 g/d in case of e.g.. tablets or capsules.

The pharmaceutical combination according to the disclosure can thus be used in the prophylaxis or treatment of a multiple sclerosis-type disease or a psoriasis-type disease, wherein the at least one fumaric acid ester or a pharmaceutically acceptable salt thereof and 5-amino-2,3-dihydro-1,4-phthalazinedione or one of its pharmaceutically acceptable salts, hydrates and solvates can be preferably used in any ratio from 1.5:1 to 1:40, more preferably from 1:1 to 1:30, still more preferably 1:3 to 1:20, most preferably from 1:5 to 1:10.

The present application refers thus to a pharmaceutical combination for use, wherein the weight ratio of the dosages of the at least one fumaric acid ester or one of its pharmaceutically acceptable salts to 5-amino-2,3-dihydro-1,4-phthalazinedione or one of its pharmaceutically acceptable salts, hydrates or solvates is in the range of 1.5:1 to 1:40.

However, in case of liquid applications higher doses and/or amounts could be administered without negative impact on patient's compliance.

Due to the supraadditivity of the pharmaceutical combinations of the invention the dose of the at least one fumaric acid ester or a pharmaceutically salt thereof can be reduced depending on individual patient, indication and ratio of components used. In some cases, dose and ratio adaption over time might become necessary to receive best results.

Thus, the dosage of the at least one fumaric acid ester or a pharmaceutically acceptable salt thereof in the pharmaceutical combination of the invention can be reduced to 80%, preferably 50%, most preferably 20% compared to the dosage of the at least one fumaric ester or a pharmaceutically acceptable salt thereof is administered alone.

The present disclosure refers likewise to 5-amino-2,3-dihydro-1,4-phthalazinedione or one of its pharmaceutically acceptable salts, hydrates or solvates for use in a method for the treatment and/or prevention of a multiple sclerosis-type disease in a subject, wherein the method comprises administering to the subject an affective amount of 5-amino-2,3-dihydro-1,4-phthalazinedione or one of its pharmaceutically acceptable salts, hydrates or solvates and concomitantly or subsequently an effective amount of at least one fumaric acid ester or one of its pharmaceutically acceptable salts.

The present disclosure refers likewise to 5-amino-2,3-dihydro-1,4-phthalazinedione or one of its pharmaceutically acceptable salts, hydrates or solvates for use in a method for the treatment and/or prevention of a psoriasis-type disease in a subject, wherein the method comprises administering to the subject an affective amount of 5-amino-2,3-dihydro-1,4-phthalazinedione or one of its pharmaceutically acceptable salts, hydrates or solvates and concomitantly or subsequently an effective amount of at least one fumaric acid ester or one of its pharmaceutically acceptable salts.

The present disclosure refers likewise to 5-amino-2,3-dihydro-1,4-phthalazinedione or one of its pharmaceutically acceptable salts, hydrates or solvates for use in a method for the treatment and/or prevention of a multiple sclerosis-type disease in a subject, wherein the method comprises administering to the subject an affective amount of 5-amino-2,3-dihydro-1,4-phthalazinedione or one of its pharmaceutically acceptable salts, hydrates or solvates and concomitantly or subsequently an effective amount of at least one fumaric acid ester or one of its pharmaceutically acceptable salts, wherein 5-amino-2,3-dihydro-1,4-phthalazinedione is a sodium salt.

The present disclosure refers likewise to 5-amino-2,3-dihydro-1,4-phthalazinedione or one of its pharmaceutically acceptable salts, hydrates or solvates for use in a method for the treatment and/or prevention of a psoriasis-type disease in a subject, wherein the method comprises administering to the subject an affective amount of 5-amino-2,3-dihydro-1,4-phthalazinedione or one of its pharmaceutically acceptable salts, hydrates or solvates and concomitantly or subsequently an effective amount of at least one fumaric acid ester or one of its pharmaceutically acceptable salts, wherein 5-amino-2,3-dihydro-1,4-phthalazinedione is a sodium salt.

The present disclosure refers likewise to 5-amino-2,3-dihydro-1,4-phthalazinedione or one of its pharmaceutically acceptable salts, hydrates or solvates for use in a method for the treatment and/or prevention of a multiple sclerosis-type disease in a subject, wherein the method comprises administering to the subject an affective amount of at least one fumaric acid ester or one of its pharmaceutically acceptable salts and concomitantly or subsequently an effective amount of 5-amino-2,3-dihydro-1,4-phthalazinedione or one of its pharmaceutically acceptable salts, hydrates or solvates.

The present disclosure refers likewise to 5-amino-2,3-dihydro-1,4-phthalazinedione or one of its pharmaceutically acceptable salts, hydrates or solvates for use in a method for the treatment and/or prevention of a psoriasis-type disease in a subject, wherein the method comprises administering to the subject an affective amount of at least one fumaric acid ester or one of its pharmaceutically acceptable salts and concomitantly or subsequently an effective amount of 5-amino-2,3-dihydro-1,4-phthalazinedione or one of its pharmaceutically acceptable salts, hydrates or solvates.

The present disclosure refers likewise to 5-amino-2,3-dihydro-1,4-phthalazinedione or one of its pharmaceutically acceptable salts, hydrates or solvates for use in a method for the treatment and/or prevention of a multiple sclerosis-type disease in a subject, wherein the method comprises administering to the subject an affective amount of at least one fumaric acid ester or one of its pharmaceutically acceptable salts and concomitantly or subsequently an effective amount of 5-amino-2,3-dihydro-1,4-phthalazinedione or one of its pharmaceutically acceptable salts, hydrates or solvates, wherein 5-amino-2,3-dihydro-1,4-phthalazinedione is a sodium salt.

The present disclosure refers likewise to 5-amino-2,3-dihydro-1,4-phthalazinedione or one of its pharmaceutically acceptable salts, hydrates or solvates for use in a method for the treatment and/or prevention of a psoriasis-type disease in a subject, wherein the method comprises administering to the subject an affective amount of at least one fumaric acid ester or one of its pharmaceutically acceptable salts and concomitantly or subsequently an effective amount of 5-amino-2,3-dihydro-1,4-phthalazinedione or one of its pharmaceutically acceptable salts, hydrates or solvates, wherein 5-amino-2,3-dihydro-1,4-phthalazinedione is a sodium salt.

In a further aspect of the invention a method of treatment is disclosed that comprises the administration of an effective dose of a pharmaceutical composition according to the disclosure for use in the treatment of a patient suffering from a multiple sclerosis-type disease or a psoriasis-type disease and in need of such a treatment.

### EXAMPLES

In all experiments, solutions containing 5-amino-2,3-dihydro-1,4-phthalazinedione sodium salt are prepared using the beforementioned anhydrous Form I of 5-amino-2,3-dihydro-1,4-phthalazinedione sodium salt (provided by MetrioPharm).

### Example 1 - MOG-induced experimental autoimmune encephalomyelitis in C57BL6/J mice

*Background:* Experimental autoimmune encephalomyelitis (EAE) originally also referred to as experimental allergic encephalomyelitis is an animal model of immunoinflammatory disease of the CNS with clinical, histological and immunological characteristics comparable to human multiple sclerosis. EAE is thus an inflammatory disease of the CNS in rodents induced by injecting a protein which causes an immune reaction. This inducible demyelinating disease shows wide similarities with human multiple sclerosis with regard to clinical, histological and immunological characteristics. This model is broadly accepted to test the comparative efficacy of pharmaceutical agents. It shows a good predictive power for their efficacy in humans. There are several varieties of this model, depending on the mouse strain and the inflammation-stimulating protein.

Herein the MOG-EAE model is used in C57BL6/J mice. Induced by the peptide pMOG₃₅₋₆₅ EAE it shows as primary progressive disease course in C57BL6/J mice.

Myelin oligodendrocyte glycoprotein (pMOG₃₅₋₅₅) - in the following referred to as MOG - is applied by subcutaneous injection. MOG is a myelin-derived protein. Its extracellular immunoglobulin-like domain is expressed on the cell membrane of oligodendrocytes (Delarasse et al. (2013) Immunology 140: 456-464). Within two weeks after immunization with an emulsion comprising MOG and complete Freund adjuvant (CFA) and challenge with pertussis toxin, peripheral immune responses are initiated that subsequently cause inflammation in the CNS. Thereafter, zones of demyelination can be seen in association with perivascular inflammation, cell infiltrates and progressively ascending paralysis. EAE induced by the MOG develops as primary-progressive form in H2b mice (i.e., C57BL6/J) and as progressive relapsing clinical form in H2U mice (Kerlero de Rosbo et al. (1995) Eur J Immunol 25: 985-993; Mendel et al. (1995) Eur J Immunol 25: 1951-1959).

*Induction:* Mice are immunized by s.c. injection of an emulsion composed of 200 µg MOG₃₅₋₅₅ peptide (Genemed Synthesis, San Francisco, CA, USA) in Complete Freund's Adjuvant (CFA; Difco, Detroit, Mi, USA) containing 0.5 mg (per injection) of *Mycobacterium tuberculosis.* Each mouse receives subcutaneous injections of 200 µl emulsion equally divided among two sites, left and right flank, draining into the axillary lymph nodes. Pertussis toxin (Calbiochem, Nottingham, UK) is used as a co-adjuvant and is administered i.p. at the dose of 200 ng/mouse on day 0 and 200 ng/mouse on day 2 post-immunization.

Due to quality aspects mice considered as SHAM are injected with Freund adjuvant only. They are not further mentioned in the following.

*Scoring:* Starting from day 7 post-immunization the animals are examined individually for the presence of paralysis according to the following score: 0 = no sign of disease; 0.5 = partial tail paralysis; 1 = tail paralysis; 1.5 = tail paralysis + partial unilateral hind limb paralysis; 2 = tail paralysis + hindlimb weakness or partial hindlimb paralysis; 2.5 = tail paralysis + partial hindlimb paralysis (lowered pelvis); 3 = tail paralysis + complete hindlimb paralysis; 3.5 = tail paralysis + complete hindlimb paralysis + incontinence; 4 = tail paralysis + hindlimb paralysis + weakness or partial paralysis of forelimbs; 5 = moribund or dead.

The following parameters are investigated in the experiments: A cumulative score is calculated for each mouse by adding the daily scores from the day of onset (score disease s 0.5) until the end of treatment. A maximal score is assessed by taking the highest score reached throughout the experiment for each animal. The disease incidence is determined by summing up the number of animals per group that ever showed a score ≥ 0.5. This parameter shows that the MOG-EAE model worked well in all experiments. Disease duration is calculated by assigning each day a score of 0 and 1 for any clinical score higher than 0 for each animal. Disease onset is determined by taking the first day an animal showed a score ≥ 0.5. Animals that never showed any symptoms are not evaluated.

Observation of the animals take place in a quiet room. Clinical signs are monitored daily and body weight is monitored three times a week in each group of treatment in blinded mode. Animals reaching a score of 3 are monitored closely and supportive care is initiated, if necessary. To these animals, soft food is offered to encourage feed intake. The loss of fluids and minerals is balanced by respective subcutaneous injections. Mice reaching a score of 4 are ethically euthanized. Also, animals with more than 20% body weight loss and/or animals experiencing severe pain or suffering are euthanized regardless of the clinical score.,

Dexamethasone (Soldesam^{®}) is used as positive control drug. In all experiments the intraperitoneal treatment with the test compound appears to be well tolerated as judged by clinical status of the mice and by body weight variation similar to vehicle-treated mice. There are no significant body weight changes throughout the groups during the whole study.

### Late prophylactic effects

*Study design:* Five groups of 10 animals each are treated with a pharmaceutical combination of 5-amino-2,3-dihydro-1,4-phthalazinedione sodium salt (Na⁺ luminolate) and dimethyl fumarate (DMF) for 40 consecutive days under a late prophylactic regime starting day 7 after immunization with intraperitoneal injections of Na⁺ luminolate and oral gavage of DMF once daily, as described in the table below.

| Group | Treatment | Daily dose |
|---|---|---|
| 1 | Na⁺ luminolate | 1.5 mg/kg |
| | DMF | 15 mg/kg |
| 2 | Na⁺ luminolate | 1.5 mg/kg |
| | DMF | 10 mg/kg |
| 3 | Na⁺ luminolate | 1.5 mg/kg |
| | DMF | 5 mg/kg |
| 4 | dexamethasone | 0.3 mg/kg |
| 5 | vehicle | - |

### Example 2: Therapeutic effects

*Study design:* Five groups of 10 animals each are used for vehicle and dexamethasone. The animals in the therapeutic regimen receive once daily i.p. injections of Na⁺ luminolate and oral gavage of DMF for 30 consecutive days, starting from the first day with a clinical score equal to or greater than 0.5.

| Group | Treatment | Daily dose |
|---|---|---|
| 1 | Na⁺ luminolate | 1.5 mg/kg |
| | DMF | 15 mg/kg |
| 2 | Na+ luminolate | 1.5 mg/kg |
| | DMF | 10 mg/kg |
| 3 | Na⁺ luminolate | 1.5 mg/kg |
| | DMF | 5 mg/kg |
| 4 | dexamethasone | 0.3 mg/kg |
| 5 | vehicle | - |

## Claims

1. Pharmaceutical combination of 5-amino-2,3-dihydro-1,4-phthalazinedione or one of its pharmaceutically acceptable salts, hydrates or solvates and at least one fumaric acid ester or a pharmaceutically acceptable salt thereof.

2. Pharmaceutical combination of 5-amino-2,3-dihydro-1,4-phthalazinedione or one of its pharmaceutically acceptable salts, hydrates or solvates and at least one fumaric acid ester or a pharmaceutically acceptable salt thereof as defined in claim 1 for use in medicine.

3. Pharmaceutical combination of 5-amino-2,3-dihydro-1,4-phthalazinedione or one of its pharmaceutically acceptable salts, hydrates or solvates and at least one fumaric acid ester or a pharmaceutically acceptable salt thereof for use according to claim 2, wherein said use in medicine is the prophylaxis or treatment of relapsing-remitting multiple sclerosis, primary progressive multiple sclerosis, secondary progressive multiple sclerosis, acute disseminated encephalomyelitis, neuromyelitis optica, idiopathic inflammatory demyelinating diseases, central pontine myelinolysis, tabes dorsalis, progressive multifocal leukoencephalopathy, acute hemorrhagic leukoencephalopathy, toxic leukoencephalopathy, leukoencephalopathy with vanishing white matter, leukoencephalopathy with neuroaxonal spheroids, reversible posterior leukoencephalopathy syndrome, megalencephalic leukoencephalopathy with subcortical cysts, hypertensive leukoencephalopathy, metachromatic leukodystrophy, Krabbe disease, Canavan disease, Alexander disease, adrenomyeloneuropathy, cerebrotendineous xanthomatosis. Pelizaeus-Merzbacher disease, hypomyelinating leukodystrophy type 7, Refsum disease, tumefactive demyelinating lesions, Baló concentric sclerosis, Marburg multiple sclerosis, Schilder's disease, solitary sclerosis, myelocortical multiple sclerosis. optic-spinal multiple sclerosis, pure spinal multiple sclerosis, LHON associated multiple sclerosis, oligoclonal band negative multiple sclerosis, oligoclonal IgM positive multiple sclerosis, Lyme disease and human immunodeficiency virus encephalopathy.

4. Pharmaceutical combination of 5-amino-2,3-dihydro-1,4-phthalazinedione or one of its pharmaceutically acceptable salts, hydrates or solvates and at least one fumaric acid ester or a pharmaceutically acceptable salt thereof for use according to claim 2, wherein said use in medicine is the prophylaxis or treatment of psoriasis, plaque psoriasis, pustular psoriasis, inverse psoriasis, napkin psoriasis, guttate psoriasis, erythrodermic psoriasis, seborrheic-like psoriasis and psoriatic arthritis.

5. Pharmaceutical combination for use according to any one of claims 2 to 4, wherein the pharmaceutically acceptable salt of 5-amino-2,3-dihydro-1,4-phthalazinedione is 5-amino-2,3-dihydro-1,4-phthalazinedione sodium salt.

6. Pharmaceutical combination for use according to any one of claims 2 to 5, wherein the at least one fumaric acid ester or one of its pharmaceutically acceptable salts is selected from a group comprising dimethyl fumarate, monomethyl fumarate, diroximel fumarate, ethyl hydrogen fumarate calcium salt, ethyl hydrogen fumarate magnesium salt, ethyl hydrogen fumarate zinc salt and mixtures thereof.

7. Pharmaceutical combination for use according to any one of claims 2 to 6, wherein 5-amino-2,3-dihydro-1,4-phthalazinedione or one of its pharmaceutically acceptable salts, hydrates or solvates is used for reducing or avoiding unwanted effects of a fumaric acid ester.

8. Pharmaceutical combination for use according to any one of claims 2 to 7, wherein said pharmaceutical combination is for use in the prophylaxis or treatment of a medical condition that is treated with at least one fumaric acid ester.

9. Pharmaceutical combination for use according to any one of claims 2 to 8, wherein the administration route is selected from intravenous, peroral, sublingual, topical, parenteral, intraperitoneal, intraarterial, intramuscular, dermal, transdermal, subcutaneous, intradermal, sublingual, intrathecal or intraventricular administration.

10. Pharmaceutical combination for use according to claim 9, wherein the administration route is peroral and a dosage form is selected from tablets, soft gelatin capsules, hard gelatin capsules, sugar-coated tablets or pills; powders or granulates; juices, syrups, drops, teas, solutions or suspensions in aqueous or non-aqueous liquids; edible foams or mousses; or in oil-in-water or water-in-oil in emulsions.

11. Pharmaceutical combination for use according to claim 9, wherein the administration route is topical and a dosage form is selected from creams, emulsions, lotions, gels, hydrogels, pastes, powders, ointments, liniment, films, dermal patches, transdermal patches, transdermal sprays or suspensions.

12. Pharmaceutical composition containing a pharmaceutical combination as defined in claim 1, a carrier and at least one pharmaceutically acceptable excipient.

13. Pharmaceutical composition according to claim 12, wherein the at least one pharmaceutically acceptable excipient is selected from a group comprising carriers, binding agents, colorants, buffers, preservatives, antioxidants, coatings, sweeteners, thickening agents, pH-regulators, acidity regulators, acidifiers, solvents, isotonizing agents, disintegrants, glidants, lubricants, emulsifiers, solubilizing agents, stabilizers, diluents, anti-caking agents (anti-adherents), permeation enhancers, sorbents, foaming agents, anti-foaming agents, opacifiers, fatliquors, consistency enhancers, hydrotropes, aromatic and flavoring substances.

14. Pharmaceutical composition according to any one of claims 12 or 13, wherein 5-amino-2,3-dihydro-1,4-phthalazinedione or one of its pharmaceutically acceptable salts, hydrates or solvates and the at least one fumaric acid ester or a pharmaceutically acceptable salt thereof are formulated in form of liposomes, micelles, multilamellar vesicles or a cyclodextrin complex.

15. A method of treatment comprising the administration of an effective dose of a pharmaceutical composition as defined in any one of claims 12 to 14 for use in the treatment of a patient in need thereof.
